# EUROPEAN PATENT SPECIFICATION

(11) **EP 3 436 151 B1**
(45) Date of publication and mention of the grant of the patent: **07.01.2026**
(21) Application number: 17776458.6
(22) Date of filing: 28.03.2017
(51) Int. Cl.: A61P 3/02, A61K 31/352, A23L 27/30, A23L 27/00

(54) **SWEETNESS AND TASTE IMPROVEMENT OF STEVIOL GLYCOSIDE OR MOGROSIDE SWEETENERS WITH FLAVONOIDS**
SÜSSE- UND GESCHMACKVERBESSERUNG VON STEVIOLGLYCOSID- ODER MOGROSIDSÜSSSTOFFEN MIT FLAVONOIDEN
AMÉLIORATION DE LA SAVEUR SUCRÉE ET DE LA SAVEUR D'ÉDULCORANTS DE GLYCOSIDES DE STÉVIOL OU DE MOGROSIDES AVEC DES FLAVONOÏDES

(30) Priority: 28.03.2016 US 201662314083 P
(43) Date of publication of application: 06.02.2019
(73) Proprietor: The Coca-Cola Company, Atlanta, GA 30313 (US)
(72) Inventor: PRAKASH, Indra, Alpharetta, GA 30022 (US); MA, Gil, Atlanta, GA 30308 (US)
(74) Representative: Dehns
(86) International application number: PCT/US2017/024538
(87) International publication number: WO 2017/172766

(56) References cited:
- EP-A1- 2 570 036
- WO-A1-2015/006764
- WO-A1-2015/015209
- WO-A1-2015/062998
- WO-A1-2015/171746
- WO-A1-2015/199169
- WO-A1-2016/023103
- US-A1- 2013 078 192
- US-A1- 2013 084 252
- US-A1- 2014 271 996
- US-A1- 2015 017 284
- US-A1- 2015 018 432
- US-A1- 2016 039 856
- US-A1- 2017 156 361
- US-B2- 9 131 716
- TOMAS-BARBERAN F A ET AL: "FLAVANONES, CHALCONES AND DIHYDROCHALCONES - NATURE, OCCORRENCE ANDDIETARY BURDEN", JOURNAL OF THE SCIENCE OF FOOD AND AGRICULTURE, WILEY & SONS, CHICHESTER, GB, vol. 80, no. 7, 15 May 2000 (2000-05-15), pages 1073 - 1080, XP000927236, ISSN: 0022-5142, DOI: 10.1002/(SICI)1097-0010(20000515)80:7<1073::AID-JSFA568>3.0.CO;2-B

## Description

### FIELD OF THE INVENTION

The present invention relates generally to beverages containing certain steviol glycoside and/or mogroside sweeteners and at least one flavonoid. The present invention further extends to methods of enhancing the sweetness of a beverage.

### BACKGROUND OF THE INVENTION

Natural caloric sugars, such as sucrose, fructose and glucose, are used to provide a pleasant taste to beverages, foods, pharmaceuticals, and oral hygienic/cosmetic products. Sucrose, in particular, imparts a taste preferred by consumers. Although sucrose provides superior sweetness characteristics, it is disadvantageously caloric.

Consumers increasingly prefer non-caloric or low caloric sweeteners have been introduced to satisfy consumer demand. However, non- and low caloric sweeteners differ from natural caloric sugars in ways that frustrate consumers. On a taste basis, non-caloric or low caloric sweeteners exhibit a temporal profile, maximal response, flavor profile, mouth feel, and/or adaptation behavior that differ from sugar. Specifically, non-caloric or low caloric sweeteners exhibit delayed sweetness onset, lingering sweet aftertaste, bitter taste, metallic taste, astringent taste, cooling taste and/or licorice-like taste. On a source basis, many non-caloric or low caloric sweeteners are synthetic chemicals. Consumer desire remains high for natural noncaloric or low caloric sweeteners that tastes like sucrose.

*Stevia rebaudiana* Bertoni is a perennial shrub of the *Asteraceae* (*Compositae*) family native to certain regions of South America. Its leaves have been used for hundreds of years in Paraguay and Brazil to sweeten local teas and medicines. The plant is commercially cultivated in Japan, Singapore, Taiwan, Malaysia, South Korea, China, Israel, India, Brazil, Australia and Paraguay.

The leaves of the plant contain a mixture containing diterpene glycosides in an amount ranging from about 10% to 15% of the total dry weight. These diterpene glycosides are about 30 to 450 times sweeter than sugar. Structurally, the diterpene glycosides are characterized by a single base, steviol, and differ by the presence of carbohydrate residues at positions C13 and C19. Typically, on a dry weight basis, the four major steviol glycosides found in the leaves of Stevia are dulcoside A (0.3%), rebaudioside C (0.6-1.0%), rebaudioside A (3.8%) and stevioside (9.1%). Other glycosides identified in Stevia extract include rebaudioside B, D, E, and F, steviolbioside and rubusoside. Among these, only stevioside and rebaudioside A are available on a commercial scale.

Mogrosides are derived from *Luo han guo,* the common name for the sweet extract made from the fruit of Siraitia grosvenorii, a herbaceous perennial vine of the *Cucurbitaceae* family native to Southern China and Northern Thailand. *Luo han guo* extracts are nearly 250 times sweeter than sugar and non-caloric. The sweetness of *Luo han guo* is generally attributed to mogrosides.

Use of steviol glycosides and mogrosides has been limited to date by certain undesirable taste properties, including licorice taste, bitterness, astringency, sweet aftertaste, bitter aftertaste and licorice aftertaste, which become more prominent at increased concentrations and impart a taste distinct from sucrose to consumables (e.g., beverages) to which they are added. In addition, maximal sweetness of most steviol glycoside and mogrosides is generally less than what is acceptable for traditional beverage formulations in sweetened consumables (e.g., beverages).

WO 2015/171746 discloses a consumable composition which includes naringenin at concentrations suitable to improve certain characteristics of the composition. US 2013/078192 discloses the use of neoflavonoids for flavor modification.

Accordingly, there remains a need for alternative sweetener systems that provide enhanced sweetness and taste properties more like sucrose-sweetened beverages.

### SUMMARY OF THE INVENTION

The present invention generally relates to flavonoid compounds useful for enhancing the sweetness, and optionally also modulating the flavour, of sweetened beverages.

The present invention provides beverages comprising at least one sweetener and at least one flavonoid, wherein the at least one sweetener is selected from a steviol glycoside blend comprising rebaudioside M in a sweetening amount and a mogroside blend comprising siamenoside I in a sweetening amount; and the at least one flavonoid is one of the following compounds:

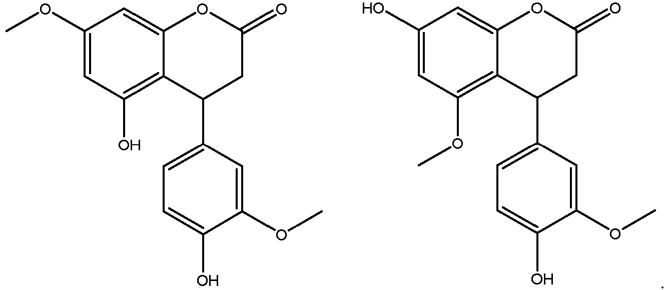

In a particular embodiment, the sweetener is a steviol glycoside blend comprising rebaudioside M in a sweetening amount. In another particular embodiment, the steviol glycoside blend further comprises rebaudioside D in a sweetening amount. In yet another particular embodiment, the sweetener is a mogroside blend comprising siamenoside I in a sweetening amount.

In one embodiment, the at least one flavonoid is present in a composition in an effective amount such that, when the composition is added to a beverage, the sucrose equivalence (SE) of the beverage is enhanced by at least about 1.2-fold (times) compared to the SE of the beverage in the absence of the at least one flavonoid.

In another embodiment, the at least one flavonoid is present a composition in an amount effective such that, when the composition is added to a beverage, one or more taste attributes of the sweetener is modulated making the beverage taste more like a sucrose- sweetened beverage compared to the beverage in the absence of the at least one flavonoid.

In one embodiment, the at least one flavonoid is present in the beverage in an amount effective to enhance the sucrose equivalence (SE) of the beverage by at least about 1.2-fold compared to the SE of the beverage in the absence of the at least one flavonoid.

In another embodiment, the at least one flavonoid is present in the beverage in an amount effective to modulate one or more taste attributes of the sweetener making the beverage taste more like a sucrose-sweetened beverage compared to the beverage in the absence of the at least one flavonoid.

In one embodiment, the at least one flavonoid is present in the beverage in a concentration from about 1 ppm to about 50 ppm.

In one embodiment, the at least one sweetener is present in the beverage in a concentration from about 50 ppm to about 600 ppm.

The beverage can be a full-calorie, mid-calorie, low-calorie or zero-calorie beverage. In certain embodiments, the beverage is a diet drink, in certain embodiments a diet cola.

The present invention also provides a method of enhancing the sweetness of a beverage comprising (i) providing a beverage comprising a sweetener described herein and (ii) adding at least one flavonoid detailed herein to the beverage to provide a beverage with enhanced sweetness. In exemplary embodiments, the SE of the beverage comprising the at least one flavonoid is enhanced by at least about 1.2-fold compared to the SE of the beverage in the absence of the at least one flavonoid.

### DETAILED DESCRIPTION OF THE INVENTION

The present invention generally relates to flavonoid compounds useful for enhancing the sweetness, and optionally also modulating the taste, of certain sweeteners in beverages. The present invention also generally relates to methods of enhancing the sweetness and/or modulating the taste of certain sweeteners in beverages, using flavonoid compounds.

### I. Definitions

"Alkyl", as used herein, generally refers to a noncyclic, cyclic, linear or branched, unsaturated or saturated hydrocarbon such as those containing from 1 to 22 carbon atoms, and specifically includes methyl, ethyl, propyl, isopropyl, cyclopropyl, butyl, isobutyl, t-butyl, pentyl, cyclopentyl, isopentyl, neopentyl, hexyl, isohexyl, cyclohexyl, cyclohexylmethyl, 3- methylpentyl, 2,2-dimethylbutyl, and 2,3-dimethylbutyl. The term includes both substituted and unsubstituted alkyl groups. Alkyl groups can be optionally substituted with one or more moieties selected from, for example, hydroxyl, amino, halo, deutero, alkylamino, arylamino, alkoxy, aryloxy, nitro, cyano, sulfonic acid, sulfate, phosphonic acid, phosphate, or phosphonate, or any other viable functional group, either unprotected, or protected, as necessary, as known to those skilled in the art, for example, as taught in T. W. Greene and P. G. M. Wuts, "Protective Groups in Organic Synthesis," 3ed., John Wiley & Sons, 1999.

The term "lower alkyl," as used herein, and unless otherwise specified, refers to a C1 to C5 saturated straight, branched, or if appropriate, a cyclic (for example, cyclopropyl) alkyl group, including both substituted and unsubstituted forms. Unless otherwise specifically stated in this application, when alkyl is a suitable moiety, lower alkyl is preferred.

"Consumables," as used herein, mean substances which are contacted with the mouth of man or animal, including substances which are taken into and subsequently ejected from the mouth and substances which are drunk, eaten, swallowed or otherwise ingested, and are safe for human or animal consumption when used in a generally acceptable range.

"Sweetness enhancer", as used herein, refers to a compound that enhances, amplifies or potentiates the perception of sweetness of a consumable (e.g. a beverage) when said compound is present in the consumable in a concentration at or below the compound's sweetener recognition threshold, i.e. a concentration at which the compound does not contribute any noticeable sweet taste in the absence of additional sweetener(s). The term "sweetness recognition threshold concentration," as generally used herein, is the lowest known concentration of a compound that is perceivable by the human sense of taste as sweet. The sweetness recognition threshold concentration is specific for a particular compound, and can vary based on temperature, matrix, ingredients and/or flavor system.

The term "sweetness enhancer" is synonymous with the terms "sweet taste potentiator," "sweetness potentiator," "sweetness amplifier," and "sweetness intensifier."

"Sweetener component", as used herein, refers to the compound or mixture of compounds that provides detectable sweetness when added to a consumable, i.e. the compound or mixture of sweet compounds present in the consumable in an amount above their sweetness recognition threshold concentration.

"Taste modulator", as used herein, refers to a compound that positively impacts the perception of a non-sucrose sweetener in a consumable (e.g. a beverage) in such a way that the consumable tastes more like a sucrose-sweetened beverage. For example, certain negative taste properties of non-sucrose sweeteners can be masked with taste modulators, e.g. bitterness, sourness, astringency, saltiness and metallic notes. In another example, mouthfeel can be improved. In still another example, sweetness linger can be decreased. In yet another example, sweetness onset can be increased. In a further example, sweetness onset can be improved. In a still further example, the bitterness linger can be improved.

### II. Enhancers/Taste Modulators

Flavonoid compounds described herein are sweetness enhancers. In one embodiment, flavonoid compounds described herein are also taste modulators, whilst simultaneously acting as sweetness enhancers. That is, in some embodiments, a flavonoid compound enhances sweetness and modulates one or more taste attributes of the sweetener. In other embodiments, a flavonoid compound enhances the sweetness of the sweetener without modulating one or more taste attributes of the sweetener.

The compounds useful as sweetness enhancers and optionally taste modulators, in the present invention are flavonoids. The flavonoid is one or more of the following compounds:

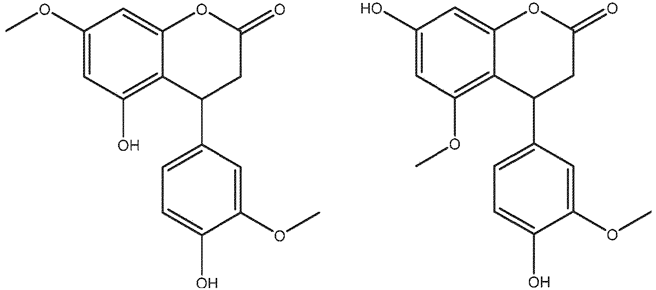

The flavonoid compounds described herein can be provided in pure form or as part of a mixture. The mixture can be an extract prepared from the plant or portion of a plant, or commercially available.

In one embodiment, the flavonoid compound comprises at least about 50% by weight of a mixture, such as, for example, at least about 60%, at least about 70%, at least about 80%, at least about 90% or at least about 95%. In a more particular embodiment, the flavonoid compound comprises at least about 96%, at least about 97%, at least about 98% or at least about 99% by weight of a mixture.

Mixtures of the two flavonoid compounds described herein are also contemplated, such as. The two flavonoid compounds can each independently be provided either in pure form or as part of a mixture. In one embodiment, the two flavonoid compounds comprise at least about 50% by weight of a mixture, such as, for example, at least about 60%, at least about 70%, at least about 80%, at least about 90% or at least about 95%. In a more particular embodiment, the two flavonoid compounds comprise at least about 96%, at least about 97%, at least about 98% or at least about 99% by weight of a mixture.

### III. Sweetener

The flavonoid compounds of the present invention enhance sweetness, and optionally modulate the taste, of certain steviol glycoside and certain mogroside sweeteners.

### A. Steviol Glycoside Sweetener

In exemplary embodiments, the sweetener is a steviol glycoside blend comprising rebaudioside M in a sweetening amount. "Sweetening amount", as used herein, refers to the amount of compound required to provide detectable sweetness when present in aa beverage.

The steviol glycoside can be natural, synthetic or a combination of natural and
synthetic.

The steviol glycoside can be provided in pure form or as part of a mixture, i.e. a steviol glycoside blend. Exemplary steviol glycoside blends further include, but are not limited to, rebaudioside D, rebaudioside A, rebaudioside N, rebaudioside O, rebaudioside E, steviolmonoside, steviolbioside, rubusoside, dulcoside B, dulcoside A, rebaudioside B, rebaudioside G, stevioside, rebaudioside C, rebaudioside F, rebaudioside I, rebaudioside H, rebaudioside L, rebaudioside K, rebaudioside J, rebaudioside M2, rebaudioside D2, rebaudioside S, rebaudioside T, rebaudioside U, rebaudioside V, rebaudioside W, rebaudioside Z1, rebaudioside Z2, rebaudioside IX, enzymatically glucosylated steviol glycosides or combinations thereof.

In certain embodiments, a steviol glycoside blend comprises at least about 5% of the steviol glycoside by weight, such as, for example, at least about 10%, at least about 20%, at least about 30%, at least about 40%, at least about 50%, at least about 60%, at least about 70%, at least about 80%, at least about 90%, at least about 95% or at least about 97%.

In exemplary embodiments, the steviol glycoside blend comprises at least about 50% of the steviol glycoside by weight, such as, for example, from about 50% to about 90%, from about 50% to about 80%, from about 50% to about 70%, from about 50% to about 60%, from about 60% to about 90%, from about 60% to about 80%, from about 60% to about 70%, from about 70% to about 90%, from about 70% to about 80% and from about 80% to about 90%.

In other exemplary embodiments, the steviol glycoside blend comprises from about 5% to about 30% of the steviol glycoside by weight, such as, for example, from about 5% to about 25%, from about 5% to about 20%, from about 5% to about 15%, from about 5% to about 10%, from about 10% to about 30%, from about 10% to about 25%, from about 10% to about 20%, from about 10% to about 15%, from about 15% to about 30%, from about 15% to about 25%, from about 15% to about 20%, from about 20% to about 30%, from about 20% to about 25% and from about 25% to about 30%.

According to the claimed invention, the sweetener is a steviol glycoside blend comprising rebaudioside M in a sweetening amount. In one embodiment, the steviol glycoside blend comprises at least about 5% rebaudioside M by weight, such as, for example, at least about 10%, at least about 20%, at least about 30%, at least about 40%, at least about 50%, at least about 60%, at least about 70%, at least about 80%, at least about 90%, at least about 95% or at least about 97%.

In exemplary embodiments, the steviol glycoside blend comprises at least about 50% rebaudioside M by weight, such as, for example, from about 50% to about 90%, from about 50% to about 80%, from about 50% to about 70%, from about 50% to about 60%, from about 60% to about 90%, from about 60% to about 80%, from about 60% to about 70%, from about 70% to about 90%, from about 70% to about 80% and from about 80% to about 90%.

In other exemplary embodiments, the steviol glycoside blend comprises from about 5% to about 30% rebaudioside M by weight, such as, for example, from about 5% to about 25%, from about 5% to about 20%, from about 5% to about 15%, from about 5% to about 10%, from about 10% to about 30%, from about 10% to about 25%, from about 10% to about 20%, from about 10% to about 15%, from about 15% to about 30%, from about 15% to about 25%, from about 15% to about 20%, from about 20% to about 30%, from about 20% to about 25% and from about 25% to about 30%.

In one embodiment, the steviol glycoside blend comprises from about 73% to about 95% rebaudioside M by weight, such as, for example, from about 73% to about 90%, from about 73% to about 85%, from about 73% to about 80%, from about 80% to about 95%, from about 80% to about 90%, from about 80% to about 85%, from about 85% to about 95%, and from about 85% to about 90%. In a particular embodiment, Reb M constitutes about 85% to about 95% of the total steviol glycoside content by weight.

In exemplary embodiments, the sweetener is a steviol glycoside blend which further comprises rebaudioside D in a sweetening amount. In one embodiment, the steviol glycoside blend comprises at least about 5% rebaudioside D by weight, such as, for example, at least about 10%, at least about 20%, at least about 30%, at least about 40%, at least about 50%, at least about 60%, at least about 70%, at least about 80%, at least about 90%, at least about 95% or at least about 97%.

In exemplary embodiments, the steviol glycoside blend further comprises at least about 50% rebaudioside D by weight, such as, for example, from about 50% to about 90%, from about 50% to about 80%, from about 50% to about 70%, from about 50% to about 60%, from about 60% to about 90%, from about 60% to about 80%, from about 60% to about 70%, from about 70% to about 90%, from about 70% to about 80% and from about 80% to about 90%.

In other exemplary embodiments, the steviol glycoside blend further comprises from about 5% to about 30% rebaudioside D by weight, such as, for example, from about 5% to about 25%, from about 5% to about 20%, from about 5% to about 15%, from about 5% to about 10%, from about 10% to about 30%, from about 10% to about 25%, from about 10% to about 20%, from about 10% to about 15%, from about 15% to about 30%, from about 15% to about 25%, from about 15% to about 20%, from about 20% to about 30%, from about 20% to about 25% and from about 25% to about 30%.

In certain embodiments, the steviol glycoside blend comprises a sweetening amount of rebaudioside M and a sweetening amount of rebaudioside D.

In other embodiments, the steviol glycoside blend sweetener typically has a total steviol glycoside content of about 95% by weight or greater on a dry basis. The remaining 5% comprises other non-steviol glycoside compounds, e.g. by-products from extraction or purification processes. In some embodiments, the steviol glycoside blend sweetener has a total steviol glycoside content of about 96% or greater, about 97% or greater, about 98% or greater or about 99% or greater. "Total steviol glycoside content", as used herein, refers to the sum of the relative weight contributions of each steviol glycoside in a sample.

In particular embodiments, rebaudioside M and rebaudioside D do not account for more than about 90% of the total steviol glycoside content by weight of the steviol glycoside blend.

In one embodiment, the steviol glycoside blend further comprises a minor component comprising rebaudioside A, rebaudioside N, rebaudioside O and rebaudioside E. These four steviol glycosides account for up to about 14% of the total steviol glycoside content of the blend by weight. For example, rebaudiosides A, N, O and E can comprise from about 1.7% to about 14% of the total steviol glycoside content by weight, from about 1.7% to about 10%, from about 1.7% to about 5%, from about 5% to about 14%, from about 5% to about 10% and from about 10% to about 14%. In a particular embodiment, rebaudiosides A, N, O and, optionally, E account for from about 5% to about 10% of the total steviol glycoside content of the blend by weight, such as, for example, from about 5% to about 8%.

Taken together, rebaudiosides D, M, A, N, O and, optionally, E can account for at least about 90% of the total steviol glycoside content of the blend by weight. In some embodiments, these steviol glycosides account for at least about 91% of the total steviol glycoside content of the blend by weight, at least about 92%, at least about 93%, at least about 94%, at least about 95%, at least about 96% or at least about 97%.

The steviol glycoside blend can further include steviol glycosides other than rebaudiosides D, M, A, N, O and, optionally, E. Exemplary steviol glycosides include, but are not limited to, e.g. steviolmonoside, steviolbioside, rubusoside, dulcoside B, dulcoside A, rebaudioside B, rebaudioside G, stevioside, rebaudioside C, rebaudioside F, rebaudioside I, rebaudioside H, rebaudioside L, rebaudioside K, rebaudioside J, rebaudioside M2, rebaudioside D2, enzymatically glucosylated steviol glycosides and combinations thereof.

In certain embodiments, the further additional rebaudioside A comprises from about 0.5% to about 4% of the total steviol glycoside content by weight, such as, for example, from about 0.5% to about 3%, from about 0.5% to about 2%, from about 0.5% to about 1%, from about 1% to about 4%, from about 1% to about 3%, from about 1% to about 2%, from about 2% to about 4%, from about 2% to about 3% and from about 3% to about 4%.

In certain embodiments, the further additional rebaudioside N comprises from about 0.5% to about 5% of the total steviol glycoside content by weight, such as, for example, from about 0.5% to about 4%, from about 0.5% to about 3%, from about 0.5% to about 2%, from about 0.5% to about 1%, from about 1% to about 5%, from about 1% to about 4, from about 1% to about 3%, from about 1% to about 2%, from about 2% to about 5%, from about 2% to about 4%, from about 2% to about 3%, from about 3% to about 5%, from about 3% to about 4%, and from about 4% to about 5%.

In certain embodiments, the further additional rebaudioside O comprises from about 0.5% to about 5% of the total steviol glycoside content by weight, such as, for example, from about 0.5% to about 4%, from about 0.5% to about 3%, from about 0.5% to about 2%, from about 0.5% to about 1%, from about 1% to about 5%, from about 1% to about 4, from about 1% to about 3%, from about 1% to about 2%, from about 2% to about 5%, from about 2% to about 4%, from about 2% to about 3%, from about 3% to about 5%, from about 3% to about 4%, and from about 4% to about 5%.

In certain embodiments, the further additional rebaudioside E comprises from about 0.2% to about 2% total steviol glycoside content by weight, such as, for example, from about 0.2% to about 1.5%, 0.2% to about 1%, 0.2% to about 0.5%, 0.5% to about 2%, 0.5% to about 1.5%, 0.5% to about 1%, 1% to about 2%, 1% to about 1.5% and about 1.5% to about 2%.

In a particular embodiment, a steviol glycoside blend comprises rebaudiosides D, M, A, N, O and E, wherein the total steviol glycoside content is about 95% or greater by weight on a dry basis.

In one embodiment, a steviol glycoside blend comprises rebaudiosides D, M, A, N, O and, optionally, E, wherein the total steviol glycoside content is about 95% or greater by weight, wherein rebaudioside D accounts for from about 55% to about 70% of the total steviol glycoside content by weight, rebaudioside M accounts for from about 18% to about 30% total steviol glycoside content by weight, rebaudioside A accounts for from about 0.5% to about 4% of the steviol glycoside content by weight, rebaudioside N accounts for from about 0.5% to about 5% of the steviol glycoside content by weight, rebaudioside O accounts for from about 0.5% to about 5% of the total steviol glycoside content by weight and, optionally, rebaudioside E accounts for from about 0.2% to about 2% total steviol glycoside content by weight.

In another embodiment, a steviol glycoside blend comprises rebaudiosides D, M, A, N, O and, optionally, E, wherein the total steviol glycoside content is about 95% or greater by weight, wherein rebaudiosides D, M, A, N, O and, optionally, E account for at least about 90% total steviol glycoside content by weight, wherein rebaudioside D accounts for from about 55% to about 70% of the total steviol glycoside content by weight, rebaudioside M accounts for from about 18% to about 30% total steviol glycoside content by weight, rebaudioside A accounts for from about 0.5% to about 4% of the steviol glycoside content by weight, rebaudioside N accounts for from about 0.5% to about 5% of the steviol glycoside content by weight, rebaudioside O accounts for from about 0.5% to about 5% of the total steviol glycoside content by weight and, optionally, rebaudioside E accounts for from about 0.2% to about 2% total steviol glycoside content by weight.

In still another embodiment, a steviol glycoside blend comprises rebaudiosides D, M, A, N, O and, optionally, E, wherein the total steviol glycoside content is about 95% or greater by weight, wherein rebaudiosides D, M, A, N, O and, optionally, E account for at least about 90% total steviol glycoside content by weight, wherein rebaudiosides D and M account for from about 80% to about 90% total steviol glycoside content by weight, rebaudioside A accounts for from about 0.5% to about 4% of the steviol glycoside content by weight, rebaudioside N accounts for from about 0.5% to about 5% of the steviol glycoside content by weight, rebaudioside O accounts for from about 0.5% to about 5% of the total steviol glycoside content by weight and, optionally, rebaudioside E accounts for from about 0.2% to about 2% total steviol glycoside content by weight.

The steviol glycoside blend can be produced by crystallization of stevia extract enriched with rebaudiosides D, M, N and O. The enriched stevia extract is crystallized from solution comprising water and at least one organic solvent. The organic solvent is selected from the group including methanol, ethanol, n-propanol, iso-propanol, butanol, acetone, or any other organic solvent known to art. In a particular embodiment the organic solvent is ethanol.

In one embodiment, the steviol glycoside blend sweetener consists essentially of rebaudioside M and rebaudioside D, wherein rebaudioside M is present in an amount from about 70% to about 90% total steviol glycoside content by weight and rebaudioside D is present in an amount from about 10% to about 20% total steviol glycoside content by weight.

The weight ratio of the at least one sweetener described hereinabove to the at least one flavonoid described herein can vary. Typically, the weight ratio of at least one sweetener to at least one flavonoid is from about 500:1 to about 2:1, such as, for example, from about 100:1 to about 2:1, from about 50:1 to about 2:1, from about 25:1 to about 2:1, from about 10:1 to about 2:1, from about 5:1 to about 2:1, from about 500:1 to about 400:1, from about 500:1 to about 300:1, from about 500:1 to about 200:1, from about 500:1 to about 100:1, from about 500:1 to about 50:1, from about 500:1 to about 25:1, from about 500:1 to about 10:1, from about 400:1 to about 300:1, from about 400:1 to about 200:1, from about 400:1 to about 100:1, from about 400:1 to about 50:1, from about 400:1 to about 25:1, from about 400:1 to about 10:1, from about 400:1 to about 6.67:1, from about 300:1 to about 200:1, from about 300:1 to about 100:1, from about 300:1 to about 50:1, from about 300:1 to about 25:1, from about 300:1 to about 10:1, from about 300:1 to about 6.67:2, from about 200:1 to about 100:1, from about 200:1 to about 50:1, from about 200:1 to about 25:1, from about 200:1 to about 10:1, from about 100:1 to about 50:1, from about 100:1 to about 25:1, from about 100:1 to about 10:1, from about 100:1 to about 6.67:1, from about 50:1 to about 25:1, from about 50:1 to about 25:1, from about 50:1 to about 10:1, from about 50:1 to about 6.65:1, from about 25:1 to about 10:1, from about 25:1 to about 6.67:1, from about 10:1 to about 6.67:1 and any range in between.

In some embodiments wherein the sweetener is a steviol glycoside blend, the weight ratio of steviol glycosides to the at least one flavonoid is greater than about 25:1, such as, for example, from about 500:1 to about 30:1, from about 500:1 to about 50:1, from about 500:1 to about 100:1, from about 500:1 to about 200:1, from about 500:1 to about 300:1, from about 500:1 to about 400:1, from about 200:1 to about 30:1, from about 200:1 to about 50:1, from about 200:1 to about 100:1, from about 100:1 to about 30:1, from about 100:1 to about 50:1, from about 75:1 to about 30:1, from about 50:1 to about 30:1.

In other embodiments wherein the sweetener is a steviol glycoside blend, the weight ratio of steviol glycosides to the at least one flavonoid is less than about 4:1, such as, for example, about 3.75:1 to about 1:1, about 3:1 to about 1:1 or about 2:1 to about 1:1.

### B. Mogroside Sweetener

In one embodiment, the sweetener is a mogroside blend comprising siamenoside I in a sweetening amount. The mogroside can be natural, synthetic or a combination of natural and synthetic.

The mogroside can be provided in pure form or as part of a mixture, i.e. a mogroside blend. Exemplary mogroside blends further include, but are not limited to, any of grosmogroside I, mogroside IA, mogroside IE, 11-oxomogroside IA, mogroside II, mogroside II A, mogroside II B, mogroside II E, 7-oxomogroside II E, mogroside III, Mogroside IIIe, 11-deoxymogroside III, mogroside IV, 11-oxomogroside IV, 11-oxomogroside IV A, mogroside V, isomogroside V, 11-deoxymogroside V, 7-oxomogroside V, 11-oxomogroside V, isomogroside V, mogroside VI, mogrol, 11-oxomogrol or combinations thereof.

Additional mogrosides include those described in U.S. Patent Application Publication 2016039864. Described mogrosides include (3β,9β,10α,11α,24R)-3-[(4-O-β-D-glucospyranosyl-6-O-β-D-glucopyranosyl]-25-hydroxyl-9-methyl-19-norlanost-5-en-24-yl-[2-O-β-D-glucopyranosyl-6-O-β-D-glucopyranosyl]-β-D-glucopyranoside); (3β, 9β, 10α, 11α, 24R)-[(2-O-β-D-glucopyranosyl-6-O-β-D-glucopyranosyl-β-D-glucopyranosyl)oxy]-25-hydroxy-9-methyl-19-norlanost-5-en-24-yl-[2-O-β-D-glucopyranosyl-6-O-β-D-glucopyranosyl]-β-D- glucopyranoside); (3β, 9β, 10α, 11α, 24R)-[(2-O-β-D-glucopyranosyl-6-O-β-D-glucopyranosyl-β-D-glucopyranosyl)oxy]-25-hydroxy-9-methyl-19-norlanost-5-en-24-yl-[2-O-β-D-glucopyranosyl-6-O-β-D-glucopyranosyl]-β-D-glucopyranoside) and combinations thereof.

The mogroside blend comprises at least one mogroside, which is siamenoside I, in a sweetening amount.

In certain embodiments, the mogroside blend comprises at least about 5% of the mogroside by weight, such as, for example, at least about 10%, at least about 20%, at least about 30%, at least about 40%, at least about 50%, at least about 60%, at least about 70%, at least about 80%, at least about 90%, at least about 95% or at least about 97%.

In exemplary embodiments, the mogroside blend comprises at least about 50% of the mogroside by weight, such as, for example, from about 50% to about 90%, from about 50% to about 80%, from about 50% to about 70%, from about 50% to about 60%, from about 60% to about 90%, from about 60% to about 80%, from about 60% to about 70%, from about 70% to about 90%, from about 70% to about 80% and from about 80% to about 90%.

In other exemplary embodiments, the mogroside blend comprises from about 5% to about 30% of the mogroside by weight, such as, for example, from about 5% to about 25%, from about 5% to about 20%, from about 5% to about 15%, from about 5% to about 10%, from about 10% to about 30%, from about 10% to about 25%, from about 10% to about 20%, from about 10% to about 15%, from about 15% to about 30%, from about 15% to about 25%, from about 15% to about 20%, from about 20% to about 30%, from about 20% to about 25% and from about 25% to about 30%.

In other embodiments, the mogroside blend has a total mogroside content of about 95% by weight or greater on a dry basis. In some embodiments, the mogroside blend sweetener has a total mogroside content of about 96% or greater, about 97% or greater, about 98% or greater or about 99% or greater. "Total mogroside content", as used herein, refers to the sum of the relative weight contributions of each mogroside in a sample.

According to the claimed invention, the sweetener is a mogroside blend comprising siamenoside I in a sweetening amount.

The weight ratio of the at least one sweetener described hereinabove to the at least one flavonoid described herein can vary. Typically, the weight ratio of at least one sweetener to at least one flavonoid is from about 500:1 to about 2:1, such as, for example, from about 100:1 to about 2:1, from about 50:1 to about 2:1, from about 25:1 to about 2:1, from about 10:1 to about 2:1, from about 5:1 to about 2:1, from about 500:1 to about 400:1, from about 500:1 to about 300:1, from about 500:1 to about 200:1, from about 500:1 to about 100:1, from about 500:1 to about 50:1, from about 500:1 to about 25:1, from about 500:1 to about 10:1, from about 400:1 to about 300:1, from about 400:1 to about 200:1, from about 400:1 to about 100:1, from about 400:1 to about 50:1, from about 400:1 to about 25:1, from about 400:1 to about 10:1, from about 400:1 to about 6.67:1, from about 300:1 to about 200:1, from about 300:1 to about 100:1, from about 300:1 to about 50:1, from about 300:1 to about 25:1, from about 300:1 to about 10:1, from about 300:1 to about 6.67:2, from about 200:1 to about 100:1, from about 200:1 to about 50:1, from about 200:1 to about 25:1, from about 200:1 to about 10:1, from about 100:1 to about 50:1, from about 100:1 to about 25:1, from about 100:1 to about 10:1, from about 100:1 to about 6.67:1, from about 50:1 to about 25:1, from about 50:1 to about 25:1, from about 50:1 to about 10:1, from about 50:1 to about 6.65:1, from about 25:1 to about 10:1, from about 25:1 to about 6.67:1, from about 10:1 to about 6.67:1 and any range in between.

In some embodiments wherein the sweetener is a mogroside blend, the weight ratio of mogrosides to the at least one flavonoid is greater than about 25:1, such as, for example, from about 500:1 to about 30:1, from about 500:1 to about 50:1, from about 500:1 to about 100:1, from about 500:1 to about 200:1, from about 500:1 to about 300:1, from about 500:1 to about 400:1, from about 200:1 to about 30:1, from about 200:1 to about 50:1, from about 200:1 to about 100:1, from about 100:1 to about 30:1, from about 100:1 to about 50:1, from about 75:1 to about 30:1, from about 50:1 to about 30:1.

In other embodiments wherein the sweetener is a mogroside blend, the weight ratio of mogrosides to the at least one flavonoid is less than about 4:1, such as, for example, about 3.75:1 to about 1:1, about 3:1 to about 1:1 or about 2:1 to about 1:1.

### III. Sweetener Component

In one embodiment, one of the sweeteners described above is the only sweetener in the beverage. In other embodiments, a beverage comprises a sweetener component containing one of the sweeteners described above and one or more additional sweeteners.

Typically, a sweetener of the present invention comprises at least about 50% by weight of the sweetener component, such as for example, at least about 60%, at least about 70%, at least about 80%, at least about 90% and at least about 95%. In a more particular embodiment, a sweetener of the present invention comprises at least about 96%, at least about 97%, at least about 98% or at least about 99% of the sweetener component.

The additional sweetener used in the sweetener component can be any known sweetener, e.g. a natural sweetener, a natural high potency sweetener, a synthetic sweetener.

As used herein, the phrase "natural high potency sweetener" refers to any sweetener found naturally in nature and characteristically has a sweetness potency greater than sucrose, fructose, or glucose, yet has less calories. The natural high potency sweetener can be provided as a pure compound or, alternatively, as part of an extract. As used herein, the phrase "synthetic sweetener" refers to any composition which is not found naturally in nature and characteristically has a sweetness potency greater than sucrose, fructose, or glucose, yet has less calories.

In other embodiments, the additional sweetener is at least one carbohydrate sweetener. Suitable carbohydrate sweeteners are selected from, but not limited to, the group consisting of sucrose, glyceraldehyde, dihydroxyacetone, erythrose, threose, erythrulose, arabinose, lyxose, ribose, xylose, ribulose, xylulose, allose, altrose, galactose, glucose, gulose, idose, mannose, talose, fructose, psicose, sorbose, tagatose, mannoheptulose, sedoheltulose, octolose, fucose, rhamnose, arabinose, turanose, sialose and combinations thereof.

Other suitable additional sweeteners include siamenoside, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, steviolbioside and cyclocarioside I, sugar alcohols such as erythritol, sucralose, potassium acesulfame, acesulfame acid and salts thereof, aspartame, alitame, saccharin and salts thereof, neohesperidin dihydrochalcone, cyclamate, cyclamic acid and salts thereof, neotame, advantame, glucosylated steviol glycosides (GSGs) and combinations thereof.

In one embodiment, the additional sweetener is a caloric sweetener or mixture of caloric sweeteners. In another embodiment, the caloric sweetener is selected from sucrose, fructose, glucose, high fructose corn/starch syrup, a beet sugar, a cane sugar, and combinations thereof.

In another embodiment, the additional sweetener is a rare sugar selected from sorbose, lyxose, ribulose, xylose, xylulose, D-allose, L-ribose, D-tagatose, L-glucose, L-fucose, L- arabinose, turanose and combinations thereof. The rare sugars can be present in the sweetener compositions in an amount from about 0.5% to about 3.0%, such as, for example, about 0.5% to about 2.5%, about 0.5% to about 2.0%, about 0.5% to about 1.5%, about 0.5% to about 1.0%, about 1.0% to about 3.0%, about 1.0% to about 2.5%, about 1.0% to about 2.0%, about 1.0% to about 1.5%, about 2.0% to about 3.0% and about 2.0% to about 2.5%.

### IV. Beverages

The present invention provides a beverage comprising at least one sweetener described hereinabove and at least one flavonoid described herein.

In one embodiment, the at least one flavonoid compound described is present in the beverage in an amount effective to enhance the sweetness thereof, and optionally modulate one or more taste attributes, of the sweetener to make the beverage taste more like a sucrose-sweetened beverage.

The at least one flavonoid compound described is present in the beverage in an amount effective to enhance the sweetness of the beverage. Typically, the at least one flavonoid is present in the beverage in an amount effective to enhance the sucrose equivalence (SE) of the beverage by at least 1.2-fold when compared to the SE of the beverage in the absence of the at least one flavonoid, such as for example, at least about 1.3-fold, at least about 1.4-fold, at least about 1.5-fold, at least about 1.6-fold, at least about 1.7-fold, at least about 1.8-fold, at least about 1.9-fold and at least about 2.0-fold.

In other embodiments, the at least one flavonoid enhances the sucrose equivalence (SE) of the beverage by at least about 2.5-fold when compared to the SE of the beverage in the absence of the at least one flavonoid, such as, for example, at least about 3.0-fold, at least about 4.0-fold or at least about 5.0-fold.

In other embodiments, the at least one flavonoid compound described is present in the beverage in an amount effective to modulate one or more taste attributes of the sweetener to make the beverage taste more like a sucrose-sweetened beverage . Exemplary taste attribute modulations include decreasing or eliminating bitterness, decreasing or eliminating bitter linger, decreasing or eliminating sourness, decreasing or eliminating astringency, decreasing or eliminating saltiness, decreasing or eliminating metallic notes, improving mouthfeel, decreasing or eliminating sweetness linger, and increasing sweetness onset. Multiple taste attributes of the sweetener can be modulated simultaneously, such that the beverage, overall, has more sucrose-sweetened characteristics. Methods of quantifying improvement in sucrose-sweetened characteristics are known in the art and includes taste testing and histogram mapping.

The particular concentration of the at least one flavonoid compound described herein and the sweetener will vary depending on the particular flavonoid(s) and sweetener(s).

In one embodiment, the at least one flavonoid described herein is present in the beverage in a concentration from about 1 ppm to about 50 ppm, such as, for example, from about 1 ppm to about 45 ppm, from about 1 ppm to about 40 ppm, from about 1 ppm to about 35 ppm, from about 1 ppm to about 30 ppm, from about 1 ppm to about 25 ppm, from about 1 ppm to about 20 ppm, from about 1 ppm to about 15 ppm, from about 1 ppm to about 10 ppm and from about 1 ppm to about 5 ppm. In another embodiment, the at least one flavonoid is present in the beverage in a concentration from about 5 ppm to about 50 ppm, from about 5 ppm to about 45 ppm, from about 5 ppm to about 40 ppm, from about 5 ppm to about 35 ppm, from about 5 ppm to about 30 ppm, from about 10 ppm to about 50 ppm, from about 10 ppm to about 40 ppm, from about 10 ppm to about 30 ppm, from about 15 ppm to about 50 ppm, from about 15 ppm to about 45 ppm, from about 15 ppm to about 40 ppm, from about 14 ppm to about 35 ppm, from about 15 ppm to about 30 ppm, from about 20 ppm to about 50 ppm, from about 20 ppm to about 40 ppm, from about 20 ppm to about 30 ppm and from about 25 ppm to about 30 ppm.

In one embodiment, the at least one sweetener described herein is present in the beverage in a concentration from about 50 ppm to about 600 ppm, such as, for example, about 50 ppm to about 500 ppm, from about 50 ppm to about 400 ppm, from about 50 ppm to about 300 ppm, from about 50 ppm to about 300 ppm, from about 50 ppm to about 200 ppm, from about 50 ppm to about 100 ppm, about 100 ppm to about 600 ppm, about 100 ppm to about 500 ppm, about 100 ppm to about 400 ppm, about 100 ppm to about 300 ppm, about 100 ppm to about 200 ppm, about 200 ppm to about 600 ppm, about 200 ppm to about 500 ppm, about 200 ppm to about 400 ppm, about 200 ppm to about 300 ppm, about 300 ppm to about 600 ppm, about 300 ppm to about 500 ppm, about 300 ppm to about 400 ppm, about 400 ppm to about 600 ppm, about 400 ppm to about 500 ppm and about 500 ppm to about 600 ppm.

The weight ratio of at least one sweetener described herein to the at least one flavonoid described herein can also vary, as discussed above.

In a particular embodiment, a beverage comprises at least one flavonoid described herein and a sweetener described herein, wherein the at least one flavonoid is present in an amount effective to enhance the SE of the beverage by at least about 1.2-fold when compared to the SE of the beverage in the absence of the at least one flavonoid.

In one embodiment, the beverage has a SE of about 2% (w/v) or greater, such as, for example, about 3% or greater, about 4% or greater, about 5% or greater, about 6% or greater, about 7% or greater, about 8% or greater, about 9% or greater, about 10% or greater, about 11% or greater, about 12% or greater, about 13% or greater or about 14% or greater.

In another embodiment, the beverage has a Brix level of about 3 to about 12, such as, for example, about 3 degrees Brix or greater, about 4 degrees Brix or greater, about 5 degrees Brix or greater, about 5 degrees Brix or greater, about 7 degrees Brix or greater, about 8 degrees Brix or greater, about 9 degrees Brix or greater, about 10 degrees Brix or greater and about 11 degrees Brix or greater. The amount of sucrose, and thus another measure of sweetness, in a reference solution may be described in degrees Brix (°Bx). One degree Brix is 1 gram of sucrose in 100 grams of solution and represents the strength of the solution as percentage by weight (% w/w) (strictly speaking, by mass).

### Beverage and Beverage Products

The consumable beverage may be a beverage product.

"Beverage product", as used herein, is a ready-to-drink beverage, a beverage concentrate, a beverage syrup, or a powdered beverage. Suitable ready-to-drink beverages include carbonated and non-carbonated beverages. Carbonated beverages include, but are not limited to, frozen carbonated beverages, enhanced sparkling beverages, cola, fruit-flavored sparkling beverages (e.g. lemon-lime, orange, grape, strawberry and pineapple), ginger-ale, soft drinks and root beer. Non-carbonated beverages include, but are not limited to, fruit juice, fruit-flavored juice, juice drinks, nectars, vegetable juice, vegetable-flavored juice, sports drinks, energy drinks, enhanced water drinks, enhanced water with vitamins, near water drinks (e.g., water with natural or synthetic flavorants), coconut water, tea type drinks (e.g. black tea, green tea, red tea, oolong tea), coffee, cocoa drink, beverage containing milk components (e.g. milk beverages, coffee containing milk components, café au lait, milk tea, fruit milk beverages), beverages containing cereal extracts and smoothies.

Beverage concentrates and beverage syrups are prepared with an initial volume of liquid matrix (e.g. water) and the desired beverage ingredients. Full strength beverages are then prepared by adding further volumes of water. Powdered beverages are prepared by dry-mixing all of the beverage ingredients in the absence of a liquid matrix. Full strength beverages are then prepared by adding the full volume of water.

Beverages comprise a matrix, i.e. the basic ingredient in which the ingredients - including the compositions of the present invention - are dissolved. In one embodiment, a beverage comprises water of beverage quality as the matrix, such as, for example deionized water, distilled water, reverse osmosis water, carbon-treated water, purified water, demineralized water and combinations thereof, can be used. Additional suitable matrices include, but are not limited to phosphoric acid, phosphate buffer, citric acid, citrate buffer and carbon-treated water.

The beverage or beverage product can further include at least one additional sweetener. Any of the sweeteners detailed herein can be used, including natural, non-natural, or synthetic sweeteners.

In one embodiment, the beverage or beverage products comprise a rare sugar - either as part of the sweetener component or added to the beverage separately. Suitable rare sugars include, but are not limited to, allulose, sorbose, lyxose, ribulose, xylose, xylulose, D-allose, L- ribose, D-tagatose, L-glucose, L-fucose, L-arabinose, turanose and combinations thereof. The rare sugars can be present in beverage in an amount from about 0.5% to about 3.0%, such as, for example, about 0.5% to about 2.5%, about 0.5% to about 2.0%, about 0.5% to about 1.5%, about 0.5% to about 1.0%, about 1.0% to about 3.0%, about 1.0% to about 2.5%, about 1.0% to about 2.0%, about 1.0% to about 1.5%, about 2.0% to about 3.0% and about 2.0% to about 2.5%. In a particular embodiment, the rare sugar is allulose.

The beverage or beverage product can contain additives including, but not limited to, carbohydrates, polyols, amino acids and their corresponding salts, poly-amino acids and their corresponding salts, sugar acids and their corresponding salts, nucleotides, organic acids, inorganic acids, organic salts including organic acid salts and organic base salts, inorganic salts, bitter compounds, caffeine, flavorants and flavoring ingredients, astringent compounds, proteins or protein hydrolysates, surfactants, emulsifiers, weighing agents, juice, dairy, cereal and other plant extracts, flavonoids, alcohols, polymers and combinations thereof. Any suitable additive described herein can be used.

The beverage or beverage product can contain one or more functional ingredients, detailed herein. Functional ingredients include, but are not limited to, vitamins, minerals, antioxidants, preservatives, glucosamine, polyphenols and combinations thereof. Any suitable functional ingredient described herein can be used.

It is contemplated that the pH of the beverage, does not materially or adversely affect the taste of the sweetener. A non-limiting example of the pH range of the beverage may be from about 1.8 to about 10. A further example includes a pH range from about 2 to about 5. In a particular embodiment, the pH of beverage can be from about 2.5 to about 4.2. On of skill in the art will understand that the pH of the beverage can vary based on the type of beverage. Dairy beverages, for example, can have pHs greater than 4.2.

The titratable acidity of a beverage may, for example, range from about 0.01 to about 1.0% by weight of beverage.

In one embodiment, the sparkling beverage product has an acidity from about 0.01 to about 1.0% by weight of the beverage, such as, for example, from about 0.05% to about 0.25% by weight of beverage.

The carbonation of a sparkling beverage product has 0 to about 2% (w/w) of carbon dioxide or its equivalent, for example, from about 0.1 to about 1.0% (w/w).

The beverage can be caffeinated or non-caffeinated.

The temperature of a beverage may, for example, range from about 4°C to about 100 °C, such as, for example, from about 4°C to about 25°C.

The beverage can be a full-calorie beverage that has up to about 120 calories per 8 oz serving.

The beverage can be a mid-calorie beverage that has up to about 60 calories per 8 oz. serving.

The beverage can be a low-calorie beverage that has up to about 40 calories per 8 oz. serving.

The beverage can be a zero-calorie that has less than about 5 calories per 8 oz.
serving.

In a particular embodiment, the beverage is a cola beverage. The cola beverage can be a low-, mid- or zero-calorie beverage.

In some embodiments, the cola beverage further comprises allulose and/or
erythritol.

In other embodiments, the cola beverage further comprises caffeine.

In a particular embodiment, a beverage comprises at least one flavonoid as described herein and a sweetener described herein above.

In one embodiment, the at least one flavonoid described herein is present in the beverage in a concentration from about 1 ppm to about 50 ppm, such as, for example, from about 1 ppm to about 45 ppm, from about 1 ppm to about 40 ppm, from about 1 ppm to about 35 ppm, from about 1 ppm to about 30 ppm, from about 1 ppm to about 25 ppm, from about 1 ppm to about 20 ppm, from about 1 ppm to about 15 ppm, from about 1 ppm to about 10 ppm and from about 1 ppm to about 5 ppm. In another embodiment, the at least one flavonoid is present in the beverage in a concentration from about 5 ppm to about 50 ppm, from about 5 ppm to about 45 ppm, from about 5 ppm to about 40 ppm, from about 5 ppm to about 35 ppm, from about 5 ppm to about 30 ppm, from about 10 ppm to about 50 ppm, from about 10 ppm to about 40 ppm, from about 10 ppm to about 30 ppm, from about 15 ppm to about 50 ppm, from about 15 ppm to about 45 ppm, from about 15 ppm to about 40 ppm, from about 14 ppm to about 35 ppm, from about 15 ppm to about 30 ppm, from about 20 ppm to about 50 ppm, from about 20 ppm to about 40 ppm, from about 20 ppm to about 30 ppm and from about 25 ppm to about 30 ppm.

In one embodiment, the at least one sweetener described herein is present in the beverage in a concentration from about 50 ppm to about 600 ppm, such as, for example, about 50 ppm to about 500 ppm, from about 50 ppm to about 400 ppm, from about 50 ppm to about 300 ppm, from about 50 ppm to about 300 ppm, from about 50 ppm to about 200 ppm, from about 50 ppm to about 100 ppm, about 100 ppm to about 600 ppm, about 100 ppm to about 500 ppm, about 100 ppm to about 400 ppm, about 100 ppm to about 300 ppm, about 100 ppm to about 200 ppm, about 200 ppm to about 600 ppm, about 200 ppm to about 500 ppm, about 200 ppm to about 400 ppm, about 200 ppm to about 300 ppm, about 300 ppm to about 600 ppm, about 300 ppm to about 500 ppm, about 300 ppm to about 400 ppm, about 400 ppm to about 600 ppm, about 400 ppm to about 500 ppm and about 500 ppm to about 600 ppm.

The weight ratio of at least one sweetener described herein to the at least one flavonoid described herein can also vary, as discussed above. The beverage can optionally include additives, functional ingredients and combinations thereof, as described herein. Any of the additives and/or functional ingredients described above can be present in the beverage.

The beverage may further comprise one or more other additives, detailed herein below. In some embodiments, the sweetener composition contains additives including, but not limited to, carbohydrates, polyols, amino acids and their corresponding salts, poly-amino acids and their corresponding salts, sugar acids and their corresponding salts, nucleotides, organic acids, inorganic acids, organic salts including organic acid salts and organic base salts, inorganic salts, bitter compounds, flavorants and flavoring ingredients, astringent compounds, proteins or protein hydrolysates, surfactants, emulsifiers, weighing agents, gums, antioxidants, colorants, additional flavonoids, alcohols, polymers and combinations thereof. In some embodiments, the additives act to improve the temporal and flavor profile to provide a taste similar to sucrose.

In one embodiment, the beverage further comprises one or more polyols. The term "polyol", as used herein, refers to a molecule that contains more than one hydroxyl group. A polyol may be a diol, triol, or a tetraol which contains 2, 3, and 4 hydroxyl groups respectively. A polyol also may contain more than 4 hydroxyl groups, such as a pentaol, hexaol, heptaol, or the like, which contain 5, 6, or 7 hydroxyl groups, respectively. Additionally, a polyol also may be a sugar alcohol, polyhydric alcohol, or polyalcohol which is a reduced form of carbohydrate, wherein the carbonyl group (aldehyde or ketone, reducing sugar) has been reduced to a primary or secondary hydroxyl group.

Non-limiting examples of polyols in some embodiments include maltitol, mannitol, sorbitol, lactitol, xylitol, isomalt, propylene glycol, glycerol (glycerin), threitol, galactitol, palatinose, reduced isomalto-oligosaccharides, reduced xylo-oligosaccharides, reduced gentio- oligosaccharides, reduced maltose syrup, reduced glucose syrup, and sugar alcohols or any other carbohydrates capable of being reduced which do not adversely affect taste.

Suitable amino acid additives include, but are not limited to, aspartic acid, arginine, glycine, glutamic acid, proline, threonine, theanine, cysteine, cystine, alanine, valine, tyrosine, leucine, arabinose, trans-4-hydroxyproline, isoleucine, asparagine, serine, lysine, histidine, ornithine, methionine, carnitine, aminobutyric acid (α-, β-, and/or δ-isomers), glutamine, hydroxyproline, taurine, norvaline, sarcosine, and their salt forms such as sodium or potassium salts or acid salts. The amino acid additives also may be in the D- or L-configuration and in the mono-, di-, or tri-form of the same or different amino acids. Additionally, the amino acids may be α-, β-, γ- and/or δ-isomers if appropriate. Combinations of the foregoing amino acids and their corresponding salts (*e.g.,* sodium, potassium, calcium, magnesium salts or other alkali or alkaline earth metal salts thereof, or acid salts) also are suitable additives in some embodiments. The amino acids may be natural or synthetic. The amino acids also may be modified. Modified amino acids refers to any amino acid wherein at least one atom has been added, removed, substituted, or combinations thereof (*e.g.,* N-alkyl amino acid, N-acyl amino acid, or N-methyl amino acid). Non-limiting examples of modified amino acids include amino acid derivatives such as trimethyl glycine, N-methylglycine, and N-methyl-alanine. As used herein, modified amino acids encompass both modified and unmodified amino acids. As used herein, amino acids also encompass both peptides and polypeptides (*e.g.,* dipeptides, tripeptides, tetrapeptides, and pentapeptides) such as glutathione and L-alanyl-L-glutamine. Suitable polyamino acid additives include poly-L-aspartic acid, poly-L-lysine (e.g., poly-L-α-lysine or poly-L-ε-lysine), poly-L-ornithine (*e.g*., poly-L-α-ornithine or poly-L-ε-ornithine), poly-L-arginine, other polymeric forms of amino acids, and salt forms thereof (*e.g.,* calcium, potassium, sodium, or magnesium salts such as L-glutamic acid mono sodium salt). The poly-amino acid additives also may be in the D- or L-configuration. Additionally, the poly-amino acids may be α-, β-, γ-, δ-, and ε-isomers if appropriate. Combinations of the foregoing poly-amino acids and their corresponding salts (*e.g.,* sodium, potassium, calcium, magnesium salts or other alkali or alkaline earth metal salts thereof or acid salts) also are suitable additives in some embodiments. The poly-amino acids described herein also may comprise co-polymers of different amino acids. The poly-amino acids may be natural or synthetic. The poly-amino acids also may be modified, such that at least one atom has been added, removed, substituted, or combinations thereof (e.g., N-alkyl polyamino acid or N-acyl poly-amino acid). As used herein, poly-amino acids encompass both modified and unmodified poly-amino acids. For example, modified poly-amino acids include, but are not limited to, poly-amino acids of various molecular weights (MW), such as poly-L-α- lysine with a MW of 1,500, MW of 6,000, MW of 25,200, MW of 63,000, MW of 83,000, or MW of 300,000.

In particular embodiments, the amino acid is present in the beverage in an amount from about 10 ppm to about 50,000 ppm. In another embodiment, the amino acid is present in the beverage in an amount from about 1,000 ppm to about 10,000 ppm, such as, for example, from about 2,500 ppm to about 5,000 ppm or from about 250 ppm to about 7,500 ppm.

Suitable sugar acid additives include, but are not limited to, aldonic, uronic, aldaric, alginic, gluconic, glucuronic, glucaric, galactaric, galacturonic, and salts thereof (e.g., sodium, potassium, calcium, magnesium salts or other physiologically acceptable salts), and combinations thereof.

Suitable nucleotide additives include, but are not limited to, inosine monophosphate ("IMP"), guanosine monophosphate ("GMP"), adenosine monophosphate ("AMP"), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, alkali or alkaline earth metal salts thereof, and combinations thereof. The nucleotides described herein also may comprise nucleotide-related additives, such as nucleosides or nucleic acid bases (*e.g.,* guanine, cytosine, adenine, thymine, uracil).

Suitable organic acid additives include any compound which comprises a -COOH moiety, such as, for example, C2-C30 carboxylic acids, substituted hydroxyl C2-C30 carboxylic acids, butyric acid (ethyl esters), substituted butyric acid (ethyl esters), benzoic acid, substituted benzoic acids (*e.g.,* 2,4-dihydroxybenzoic acid), substituted cinnamic acids, hydroxyacids, substituted hydroxybenzoic acids, anisic acid substituted cyclohexyl carboxylic acids, tannic acid, aconitic acid, lactic acid, tartaric acid, citric acid, isocitric acid, gluconic acid, glucoheptonic acids, adipic acid, hydroxycitric acid, malic acid, fruitaric acid (a blend of malic, fumaric, and tartaric acids), fumaric acid, maleic acid, succinic acid, chlorogenic acid, salicylic acid, creatine, caffeic acid, bile acids, acetic acid, ascorbic acid, alginic acid, erythorbic acid, polyglutamic acid, glucono delta lactone, and their alkali or alkaline earth metal salt derivatives thereof. In addition, the organic acid additives also may be in either the D- or L-configuration.

Suitable organic acid additive salts include, but are not limited to, sodium, calcium, potassium, and magnesium salts of all organic acids, such as salts of citric acid, malic acid, tartaric acid, fumaric acid, lactic acid (*e.g.,* sodium lactate), alginic acid (*e.g.,* sodium alginate), ascorbic acid (*e.g.,* sodium ascorbate), benzoic acid (*e.g.,* sodium benzoate or potassium benzoate), sorbic acid and adipic acid. The examples of the organic acid additives described optionally may be substituted with at least one group chosen from hydrogen, alkyl, alkenyl, alkynyl, halo, haloalkyl, carboxyl, acyl, acyloxy, amino, amido, carboxyl derivatives, alkylamino, dialkylamino, arylamino, alkoxy, aryloxy, nitro, cyano, sulfo, thiol, imine, sulfonyl, sulfenyl, sulfinyl, sulfamyl, carboxalkoxy, carboxamido, phosphonyl, phosphinyl, phosphoryl, phosphino, thioester, thioether, anhydride, oximino, hydrazino, carbamyl, phosphor or phosphonato. In particular embodiments, the organic acid additive is present in the sweetener composition in an amount effective to provide a concentration from about 10 ppm to about 5,000 ppm when present in a beverage.

Suitable inorganic acid additives include, but are not limited to, phosphoric acid, phosphorous acid, polyphosphoric acid, hydrochloric acid, sulfuric acid, carbonic acid, sodium dihydrogen phosphate, and alkali or alkaline earth metal salts thereof (*e.g.,* inositol hexaphosphate Mg/Ca).

The inorganic acid additive is present in the beverage in a concentration from about 25 ppm to about 25,000 ppm.

Suitable bitter compound additives include, but are not limited to, caffeine, quinine, urea, bitter orange oil, naringin, quassia, and salts thereof.

The bitter compound is present in the beverage in a concentration from about 25 ppm to about 25,000 ppm.

Suitable flavorants and flavoring ingredient additives include, but are not limited to, vanillin, vanilla extract, mango extract, cinnamon, citrus, coconut, ginger, viridiflorol, almond, menthol (including menthol without mint), grape skin extract, and grape seed extract. "Flavorant" and "flavoring ingredient" are synonymous and can include natural or synthetic substances or combinations thereof. Flavorants also include any other substance which imparts flavor and may include natural or non-natural (synthetic) substances which are safe for human or animals when used in a generally accepted range. Non-limiting examples of proprietary flavorants include Döhler^{™} Natural Flavoring Sweetness Enhancer K14323 (Döhler^{™}, Darmstadt, Germany), Symrise^{™} Natural Flavor Mask for Sweeteners 161453 and 164126 (Symrise^{™}, Holzminden, Germany), Natural Advantage^{™} Bitterness Blockers 1, 2, 9 and 10 (Natural Advantage^{™}, Freehold, New Jersey, U.S.A.), and Sucramask^{™} (Creative Research Management, Stockton, California, U.S.A.).

The flavorant is present in the beverage in a concentration from about 0.1 ppm to about 4,000 ppm.

Suitable polymer additives include, but are not limited to, chitosan, pectin, pectic, pectinic, polyuronic, polygalacturonic acid, starch, food hydrocolloid or crude extracts thereof (e.g., gum acacia senegal (Fibergum^{™}), gum acacia seyal, carageenan), poly-L-lysine (e.g., poly-L-α-lysine or poly-L-ε-lysine), poly-L-ornithine (e.g., poly-L-α-ornithine or poly-L-ε-ornithine), polypropylene glycol, polyethylene glycol, poly(ethylene glycol methyl ether), polyarginine, polyaspartic acid, polyglutamic acid, polyethylene imine, alginic acid, sodium alginate, propylene glycol alginate, and sodium polyethyleneglycolalginate, sodium hexametaphosphate and its salts, and other cationic polymers and anionic polymers.

The polymer is present in the beverage a concentration from about 30 ppm to about 2,000 ppm.

Suitable protein or protein hydrolysate additives include, but are not limited to, bovine serum albumin (BSA), whey protein (including fractions or concentrates thereof such as 90% instant whey protein isolate, 34% whey protein, 50% hydrolyzed whey protein, and 80% whey protein concentrate), soluble rice protein, soy protein, protein isolates, protein hydrolysates, reaction products of protein hydrolysates, glycoproteins, and/or proteoglycans containing amino acids (e.g., glycine, alanine, serine, threonine, asparagine, glutamine, arginine, valine, isoleucine, leucine, norvaline, methionine, proline, tyrosine, hydroxyproline, and the like), collagen (e.g., gelatin), partially hydrolyzed collagen (e.g., hydrolyzed fish collagen), and collagen hydrolysates (e.g., porcine collagen hydrolysate).

The protein hydrolysate is present in the beverage in a concentration from about 200 ppm to about 50,000 ppm.

Suitable surfactant additives include, but are not limited to, polysorbates (e.g., polyoxyethylene sorbitan monooleate (polysorbate 80), polysorbate 20, polysorbate 60), sodium dodecylbenzenesulfonate, dioctyl sulfosuccinate or dioctyl sulfosuccinate sodium, sodium dodecyl sulfate, cetylpyridinium chloride (hexadecylpyridinium chloride), hexadecyltrimethylammonium bromide, sodium cholate, carbamoyl, choline chloride, sodium glycocholate, sodium taurodeoxycholate, lauric arginate, sodium stearoyl lactylate, sodium taurocholate, lecithins, sucrose oleate esters, sucrose stearate esters, sucrose palmitate esters, sucrose laurate esters, and other emulsifiers, and the like.

The surfactant additive is present in the beverage in a concentration from about 30 ppm to about 2,000 ppm.

Suitable flavonoid additives are classified as flavonols, flavones, flavanones, flavan-3- ols, isoflavones, or anthocyanidins. Non-limiting examples of flavonoid additives include, but are not limited to, catechins (e.g., green tea extracts such as Polyphenon^{™} 60, Polyphenon^{™} 30, and Polyphenon^{™} 25 (Mitsui Norin Co., Ltd., Japan), polyphenols, rutins (e.g., enzyme modified rutin Sanmelin^{™} AO (San-fi Gen F.F.I., Inc., Osaka, Japan)), neohesperidin, naringin, neohesperidin dihydrochalcone, and the like.

The flavonoid additive is present in the beverage in a concentration from about 0.1 ppm to about 1,000 ppm.

Suitable alcohol additives include, but are not limited to, ethanol. In particular embodiments, the alcohol additive is present in the beverage in a concentration from about 625 ppm to about 10,000 ppm.

Suitable astringent compound additives include, but are not limited to, tannic acid, europium chloride (EuCl₃), gadolinium chloride (GdCl₃), terbium chloride (TbCl₃), alum, tannic acid, and polyphenols (e.g., tea polyphenols). The astringent additive is present in the beverage in a concentration from about 10 ppm to about 5,000 ppm.

### Functional Ingredients

The beverages provided herein can also contain one or more functional ingredients, which provide a real or perceived heath benefit to the composition. Functional ingredients include, but are not limited to, saponins, antioxidants, dietary fiber sources, fatty acids, vitamins, glucosamine, minerals, preservatives, hydration agents, probiotics, prebiotics, weight management agents, osteoporosis management agents, phytoestrogens, long chain primary aliphatic saturated alcohols, phytosterols and combinations thereof.

### Saponin

In certain embodiments, the functional ingredient is at least one saponin. As used herein, the at least one saponin may comprise a single saponin or a plurality of saponins as a functional ingredient for the composition provided herein. Generally, according to particular embodiments of this invention, the at least one saponin is present in the composition in an amount sufficient to promote health and wellness.

Saponins are glycosidic natural plant products comprising an aglycone ring structure and one or more sugar moieties. The combination of the nonpolar aglycone and the water soluble sugar moiety gives saponins surfactant properties, which allow them to form a foam when shaken in an aqueous solution.

The saponins are grouped together based on several common properties. **In** particular, saponins are surfactants which display hemolytic activity and form complexes with cholesterol. Although saponins share these properties, they are structurally diverse. The types of aglycone ring structures forming the ring structure in saponins can vary greatly. Non-limiting examples of the types of aglycone ring structures in saponin for use in particular embodiments of the invention include steroids, triterpenoids, and steroidal alkaloids. Non-limiting examples of specific aglycone ring structures for use in particular embodiments of the invention include soyasapogenol A, soyasapogenol B and soyasopogenol E. The number and type of sugar moieties attached to the aglycone ring structure can also vary greatly. Non-limiting examples of sugar moieties for use in particular embodiments of the invention include glucose, galactose, glucuronic acid, xylose, rhamnose, and methylpentose moieties. Non-limiting examples of specific saponins for use in particular embodiments of the invention include group A acetyl saponin, group B acetyl saponin, and group E acetyl saponin.

Saponins can be found in a large variety of plants and plant products, and are especially prevalent in plant skins and barks where they form a waxy protective coating. Several common sources of saponins include soybeans, which have approximately 5% saponin content by dry weight, soapwort plants (*Saponaria*), the root of which was used historically as soap, as well as alfalfa, aloe, asparagus, grapes, chickpeas, yucca, and various other beans and weeds. Saponins may be obtained from these sources by using extraction techniques well known to those of ordinary skill in the art. A description of conventional extraction techniques can be found in U.S. Pat. Appl. No. 2005/0123662.

### Antioxidant

In certain embodiments, the functional ingredient is at least one antioxidant. As used herein, the at least one antioxidant may comprise a single antioxidant or a plurality of antioxidants as a functional ingredient for the compositions provided herein. Generally, according to particular embodiments of this invention, the at least one antioxidant is present in the composition in an amount sufficient to promote health and wellness.

As used herein "antioxidant" refers to any substance which inhibits, suppresses, or reduces oxidative damage to cells and biomolecules. Without being bound by theory, it is believed that antioxidants inhibit, suppress, or reduce oxidative damage to cells or biomolecules by stabilizing free radicals before they can cause harmful reactions. As such, antioxidants may prevent or postpone the onset of some degenerative diseases.

Examples of suitable antioxidants for embodiments of this invention include, but are not limited to, vitamins, vitamin cofactors, minerals, hormones, carotenoids, carotenoid terpenoids, non-carotenoid terpenoids, flavonoids, flavonoid polyphenolics (e.g., bioflavonoids), flavonols, flavones, phenols, polyphenols, esters of phenols, esters of polyphenols, nonflavonoid phenolics, isothiocyanates, and combinations thereof. In some embodiments, the antioxidant is vitamin A, vitamin C, vitamin E, ubiquinone, mineral selenium, manganese, melatonin, α-carotene, β- carotene, lycopene, lutein, zeanthin, crypoxanthin, reservatol, eugenol, quercetin, catechin, gossypol, hesperetin, curcumin, ferulic acid, thymol, hydroxytyrosol, tumeric, thyme, olive oil, lipoic acid, glutathinone, gutamine, oxalic acid, tocopherol-derived compounds, butylated hydroxyanisole (BHA), butylated hydroxytoluene (BHT), ethylenediaminetetraacetic acid (EDTA), tert-butylhydroquinone, acetic acid, pectin, tocotrienol, tocopherol, coenzyme Q10, zeaxanthin, astaxanthin, canthaxantin, saponins, limonoids, kaempfedrol, myricetin, isorhamnetin, proanthocyanidins, quercetin, rutin, luteolin, apigenin, tangeritin, hesperetin, naringenin, erodictyol, flavan-3-ols (e.g., anthocyanidins), gallocatechins, epicatechin and its gallate forms, epigallocatechin and its gallate forms (ECGC) theaflavin and its gallate forms, thearubigins, isoflavone, phytoestrogens, genistein, daidzein, glycitein, anythocyanins, cyaniding, delphinidin, malvidin, pelargonidin, peonidin, petunidin, ellagic acid, gallic acid, salicylic acid, rosmarinic acid, cinnamic acid and its derivatives (e.g., ferulic acid), chlorogenic acid, chicoric acid, gallotannins, ellagitannins, anthoxanthins, betacyanins and other plant pigments, silymarin, citric acid, lignan, antinutrients, bilirubin, uric acid, R-□-lipoic acid, N- acetylcysteine, emblicanin, apple extract, apple skin extract (applephenon), rooibos extract red, rooibos extract, green, hawthorn berry extract, red raspberry extract, green coffee antioxidant (GCA), aronia extract 20%, grape seed extract (VinOseed), cocoa extract, hops extract, mangosteen extract, mangosteen hull extract, cranberry extract, pomegranate extract, pomegranate hull extract, pomegranate seed extract, hawthorn berry extract, pomella pomegranate extract, cinnamon bark extract, grape skin extract, bilberry extract, pine bark extract, pycnogenol, elderberry extract, mulberry root extract, wolfberry (gogi) extract, blackberry extract, blueberry extract, blueberry leaf extract, raspberry extract, turmeric extract, citrus bioflavonoids, black currant, ginger, acai powder, green coffee bean extract, green tea extract, and phytic acid, or combinations thereof. In alternate embodiments, the antioxidant is a synthetic antioxidant such as butylated hydroxytolune or butylated hydroxyanisole, for example. Other sources of suitable antioxidants for embodiments of this invention include, but are not limited to, fruits, vegetables, tea, cocoa, chocolate, spices, herbs, rice, organ meats from livestock, yeast, whole grains, or cereal grains.

Particular antioxidants belong to the class of phytonutrients called polyphenols (also known as "polyphenolics"), which are a group of chemical substances found in plants, characterized by the presence of more than one phenol group per molecule. A variety of health benefits may be derived from polyphenols, including prevention of cancer, heart disease, and chronic inflammatory disease and improved mental strength and physical strength, for example. Suitable polyphenols for embodiments of this invention include catechins, proanthocyanidins, procyanidins, anthocyanins, quercerin, rutin, reservatrol, isoflavones, curcumin, punicalagin, ellagitannin, hesperidin, naringin, citrus flavonoids, chlorogenic acid, other similar materials, and combinations thereof.

In particular embodiments, the antioxidant is a catechin such as, for example, epigallocatechin gallate (EGCG). Suitable sources of catechins for embodiments of this invention include, but are not limited to, green tea, white tea, black tea, oolong tea, chocolate, cocoa, red wine, grape seed, red grape skin, purple grape skin, red grape juice, purple grape juice, berries, pycnogenol, and red apple peel.

In some embodiments, the antioxidant is chosen from proanthocyanidins, procyanidins or combinations thereof. Suitable sources of proanthocyanidins and procyanidins for embodiments of this invention include, but are not limited to, red grapes, purple grapes, cocoa, chocolate, grape seeds, red wine, cacao beans, cranberry, apple peel, plum, blueberry, black currants, choke berry, green tea, sorghum, cinnamon, barley, red kidney bean, pinto bean, hops, almonds, hazelnuts, pecans, pistachio, pycnogenol, and colorful berries.

In particular embodiments, the antioxidant is an anthocyanin. Suitable sources of anthocyanins for embodiments of this invention include, but are not limited to, red berries, blueberries, bilberry, cranberry, raspberry, cherry, pomegranate, strawberry, elderberry, choke berry, red grape skin, purple grape skin, grape seed, red wine, black currant, red currant, cocoa, plum, apple peel, peach, red pear, red cabbage, red onion, red orange, and blackberries.

In some embodiments, the antioxidant is chosen from quercetin, rutin or combinations thereof. Suitable sources of quercetin and rutin for embodiments of this invention include, but are not limited to, red apples, onions, kale, bog whortleberry, lingonberrys, chokeberry, cranberry, blackberry, blueberry, strawberry, raspberry, black currant, green tea, black tea, plum, apricot, parsley, leek, broccoli, chili pepper, berry wine, and ginkgo.

In some embodiments, the antioxidant is reservatrol. Suitable sources of reservatrol for embodiments of this invention include, but are not limited to, red grapes, peanuts, cranberry, blueberry, bilberry, mulberry, Japanese Itadori tea, and red wine.

In particular embodiments, the antioxidant is an isoflavone. Suitable sources of isoflavones for embodiments of this invention include, but are not limited to, soy beans, soy products, legumes, alfalfa sprouts, chickpeas, peanuts, and red clover.

In some embodiments, the antioxidant is curcumin. Suitable sources of curcumin for embodiments of this invention include, but are not limited to, turmeric and mustard.

In particular embodiments, the antioxidant is chosen from punicalagin, ellagitannin or combinations thereof. Suitable sources of punicalagin and ellagitannin for embodiments of this invention include, but are not limited to, pomegranate, raspberry, strawberry, walnut, and oakaged red wine.

In particular embodiments, the antioxidant is chlorogenic acid. Suitable sources of chlorogenic acid for embodiments of this invention include, but are not limited to, green coffee, yerba mate, red wine, grape seed, red grape skin, purple grape skin, red grape juice, purple grape juice, apple juice, cranberry, pomegranate, blueberry, strawberry, sunflower, Echinacea, pycnogenol, and apple peel.

### Dietary Fiber

In certain embodiments, the functional ingredient is at least one dietary fiber source. As used herein, the at least one dietary fiber source may comprise a single dietary fiber source or a plurality of dietary fiber sources as a functional ingredient for the compositions provided herein. Generally, according to particular embodiments of this invention, the at least one dietary fiber source is present in the composition in an amount sufficient to promote health and wellness.

Numerous polymeric carbohydrates having significantly different structures in both composition and linkages fall within the definition of dietary fiber. Such compounds are well known to those skilled in the art, non-limiting examples of which include non-starch polysaccharides, lignin, cellulose, methylcellulose, the hemicelluloses, β-glucans, pectins, gums, mucilage, waxes, inulins, oligosaccharides, fructooligosaccharides, cyclodextrins, chitins, and combinations thereof.

Polysaccharides are complex carbohydrates composed of monosaccharides joined by glycosidic linkages. Non-starch polysaccharides are bonded with β-linkages, which humans are unable to digest due to a lack of an enzyme to break the β-linkages. Conversely, digestible starch polysaccharides generally comprise α(1-4) linkages.

Lignin is a large, highly branched and cross-linked polymer based on oxygenated phenylpropane units. Cellulose is a linear polymer of glucose molecules joined by a β(1-4) linkage, which mammalian amylases are unable to hydrolyze. Methylcellulose is a methyl ester of cellulose that is often used in foodstuffs as a thickener, and emulsifier. It is commercially available (e.g., Citrucel by GlaxoSmithKline, Celevac by Shire Pharmaceuticals). Hemicelluloses are highly branched polymers consisting mainly of glucurono- and 4-O- methylglucuroxylans. β-Glucans are mixed-linkage (1-3), (1-4) β-D-glucose polymers found primarily in cereals, such as oats and barley. Pectins, such as beta pectin, are a group of polysaccharides composed primarily of D-galacturonic acid, which is methoxylated to variable degrees.

Gums and mucilages represent a broad array of different branched structures. Guar gum, derived from the ground endosperm of the guar seed, is a galactomannan. Guar gum is commercially available (e.g., Benefiber by Novartis AG). Other gums, such as gum arabic and pectins, have still different structures. Still other gums include xanthan gum, gellan gum, tara gum, psylium seed husk gum, and locust been gum.

Waxes are esters of ethylene glycol and two fatty acids, generally occurring as a hydrophobic liquid that is insoluble in water.

Inulins comprise naturally occurring oligosaccharides belonging to a class of carbohydrates known as fructans. They generally are comprised of fructose units joined by β(2- 1) glycosidic linkages with a terminal glucose unit. Oligosaccharides are saccharide polymers containing typically three to six component sugars. They are generally found either O- or N- linked to compatible amino acid side chains in proteins or to lipid molecules. Fructooligosaccharides are oligosaccharides consisting of short chains of fructose molecules.

Food sources of dietary fiber include, but are not limited to, grains, legumes, fruits, and vegetables. Grains providing dietary fiber include, but are not limited to, oats, rye, barley, wheat,. Legumes providing fiber include, but are not limited to, peas and beans such as soybeans. Fruits and vegetables providing a source of fiber include, but are not limited to, apples, oranges, pears, bananas, berries, tomatoes, green beans, broccoli, cauliflower, carrots, potatoes, celery. Plant foods such as bran, nuts, and seeds (such as flax seeds) are also sources of dietary fiber. Parts of plants providing dietary fiber include, but are not limited to, the stems, roots, leaves, seeds, pulp, and skin.

Although dietary fiber generally is derived from plant sources, indigestible animal products such as chitins are also classified as dietary fiber. Chitin is a polysaccharide composed of units of acetylglucosamine joined by β(1-4) linkages, similar to the linkages of cellulose.

Sources of dietary fiber often are divided into categories of soluble and insoluble fiber based on their solubility in water. Both soluble and insoluble fibers are found in plant foods to varying degrees depending upon the characteristics of the plant. Although insoluble in water, insoluble fiber has passive hydrophilic properties that help increase bulk, soften stools, and shorten transit time of fecal solids through the intestinal tract.

Unlike insoluble fiber, soluble fiber readily dissolves in water. Soluble fiber undergoes active metabolic processing via fermentation in the colon, increasing the colonic microflora and thereby increasing the mass of fecal solids. Fermentation of fibers by colonic bacteria also yields end-products with significant health benefits. For example, fermentation of the food masses produces gases and short-chain fatty acids. Acids produced during fermentation include butyric, acetic, propionic, and valeric acids that have various beneficial properties such as stabilizing blood glucose levels by acting on pancreatic insulin release and providing liver control by glycogen breakdown. In addition, fiber fermentation may reduce atherosclerosis by lowering cholesterol synthesis by the liver and reducing blood levels of LDL and triglycerides. The acids produced during fermentation lower colonic pH, thereby protecting the colon lining from cancer polyp formation. The lower colonic pH also increases mineral absorption, improves the barrier properties of the colonic mucosal layer, and inhibits inflammatory and adhesion irritants.

Fermentation of fibers also may benefit the immune system by stimulating production of T-helper cells, antibodies, leukocytes, splenocytes, cytokinins and lymphocytes.

### Fatty Acid

In certain embodiments, the functional ingredient is at least one fatty acid. As used herein, the at least one fatty acid may be single fatty acid or a plurality of fatty acids as a functional ingredient for the compositions provided herein. Generally, according to particular embodiments of this invention, the at least one fatty acid is present in the composition in an amount sufficient to promote health and wellness.

As used herein, "fatty acid" refers to any straight chain monocarboxylic acid and includes saturated fatty acids, unsaturated fatty acids, long chain fatty acids, medium chain fatty acids, short chain fatty acids, fatty acid precursors (including omega-9 fatty acid precursors), and esterified fatty acids. As used herein, "long chain polyunsaturated fatty acid" refers to any polyunsaturated carboxylic acid or organic acid with a long aliphatic tail. As used herein, "omega-3 fatty acid" refers to any polyunsaturated fatty acid having a first double bond as the third carbon-carbon bond from the terminal methyl end of its carbon chain. In particular embodiments, the omega-3 fatty acid may comprise a long chain omega-3 fatty acid. As used herein, "omega-6 fatty acid" any polyunsaturated fatty acid having a first double bond as the sixth carbon-carbon bond from the terminal methyl end of its carbon chain.

Suitable omega-3 fatty acids for use in embodiments of the present invention can be derived from algae, fish, animals, plants, or combinations thereof, for example. Examples of suitable omega-3 fatty acids include, but are not limited to, linolenic acid, alpha-linolenic acid, eicosapentaenoic acid, docosahexaenoic acid, stearidonic acid, eicosatetraenoic acid and combinations thereof. In some embodiments, suitable omega-3 fatty acids can be provided in fish oils, (e.g., menhaden oil, tuna oil, salmon oil, bonito oil, and cod oil), microalgae omega-3 oils or combinations thereof. In particular embodiments, suitable omega-3 fatty acids may be derived from commercially available omega-3 fatty acid oils such as Microalgae DHA oil (from Martek, Columbia, MD), OmegaPure (from Omega Protein, Houston, TX), Marinol C-38 (from Lipid Nutrition, Channahon, IL), Bonito oil and MEG-3 (from Ocean Nutrition, Dartmouth, NS), Evogel (from Symrise, Holzminden, Germany), Marine Oil, from tuna or salmon (from Arista Wilton, CT), OmegaSource 2000, Marine Oil, from menhaden and Marine Oil, from cod (from OmegaSource, RTP, NC).

Suitable omega-6 fatty acids include, but are not limited to, linoleic acid, gamma-linolenic acid, dihommo-gamma-linolenic acid, arachidonic acid, eicosadienoic acid, docosadienoic acid, adrenic acid, docosapentaenoic acid and combinations thereof.

Suitable esterified fatty acids for embodiments of the present invention may include, but are not limited to, monoacylgycerols containing omega-3 and/or omega-6 fatty acids, diacylgycerols containing omega-3 and/or omega-6 fatty acids, or triacylgycerols containing omega-3 and/or omega-6 fatty acids and combinations thereof.

### Vitamin

In certain embodiments, the functional ingredient is at least one vitamin.

As used herein, the at least one vitamin may be single vitamin or a plurality of vitamins as a functional ingredient for the compositions provided herein. Generally, according to particular embodiments of this invention, the at least one vitamin is present in the composition in an amount sufficient to promote health and wellness.

Vitamins are organic compounds that the human body needs in small quantities for normal functioning. The body uses vitamins without breaking them down, unlike other nutrients such as carbohydrates and proteins. To date, thirteen vitamins have been recognized, and one or more can be used in the compositions herein. Suitable vitamins include, vitamin A, vitamin D, vitamin E, vitamin K, vitamin B1, vitamin B2, vitamin B3, vitamin B5, vitamin B6, vitamin B7, vitamin B9, vitamin B12, and vitamin C.

Various other compounds have been classified as vitamins by some authorities. These compounds may be termed pseudo-vitamins and include, but are not limited to, compounds such as ubiquinone (coenzyme Q10), pangamic acid, dimethylglycine, taestrile, amygdaline, flavanoids, para-aminobenzoic acid, adenine, adenylic acid, and s-methylmethionine. As used herein, the term vitamin includes pseudo-vitamins.

In some embodiments, the vitamin is a fat-soluble vitamin chosen from vitamin A, D, E, K and combinations thereof.

In other embodiments, the vitamin is a water-soluble vitamin chosen from vitamin B1, vitamin B2, vitamin B3, vitamin B6, vitamin B12, folic acid, biotin, pantothenic acid, vitamin C and combinations thereof.

### Glucosamine

In certain embodiments, the functional ingredient is glucosamine.

Generally, according to particular embodiments of this invention, glucosamine is present in the compositions in an amount sufficient to promote health and wellness.

Glucosamine, also called chitosamine, is an amino sugar that is believed to be an important precursor in the biochemical synthesis of glycosylated proteins and lipids. D-glucosamine occurs naturally in the cartilage in the form of glucosamine-6-phosphate, which is synthesized from fructose-6-phosphate and glutamine. However, glucosamine also is available in other forms, non-limiting examples of which include glucosamine hydrochloride, glucosamine sulfate, N-acetyl-glucosamine, or any other salt forms or combinations thereof. Glucosamine may be obtained by acid hydrolysis of the shells of lobsters, crabs, shrimps, or prawns using methods well known to those of ordinary skill in the art. In a particular embodiment, glucosamine may be derived from fungal biomass containing chitin, as described in U.S. Patent Publication No. 2006/0172392.

The compositions can further comprise chondroitin sulfate.

### Mineral

In certain embodiments, the functional ingredient is at least one mineral.

As used herein, the at least one mineral may be single mineral or a plurality of minerals as a functional ingredient for the compositions provided herein. Generally, according to particular embodiments of this invention, the at least one mineral is present in the composition in an amount sufficient to promote health and wellness.

Minerals, in accordance with the teachings of this invention, comprise inorganic chemical elements required by living organisms. Minerals are comprised of a broad range of compositions (e.g., elements, simple salts, and complex silicates) and also vary broadly in crystalline structure. They may naturally occur in foods and beverages, may be added as a supplement, or may be consumed or administered separately from foods or beverages.

Minerals may be categorized as either bulk minerals, which are required in relatively large amounts, or trace minerals, which are required in relatively small amounts. Bulk minerals generally are required in amounts greater than or equal to about 100 mg per day and trace minerals are those that are required in amounts less than about 100 mg per day.

In particular embodiments of this invention, the mineral is chosen from bulk minerals, trace minerals or combinations thereof. Non-limiting examples of bulk minerals include calcium, chlorine, magnesium, phosphorous, potassium, sodium, and sulfur. Non-limiting examples of trace minerals include chromium, cobalt, copper, fluorine, iron, manganese, molybdenum, selenium, zinc, and iodine. Although iodine generally is classified as a trace mineral, it is required in larger quantities than other trace minerals and often is categorized as a bulk mineral.

In other particular embodiments of this invention, the mineral is a trace mineral, believed to be necessary for human nutrition, non-limiting examples of which include bismuth, boron, lithium, nickel, rubidium, silicon, strontium, tellurium, tin, titanium, tungsten, and vanadium.

The minerals embodied herein may be in any form known to those of ordinary skill in the art. For example, in a particular embodiment the minerals may be in their ionic form, having either a positive or negative charge. In another particular embodiment the minerals may be in their molecular form. For example, sulfur and phosphorous often are found naturally as sulfates, sulfides, and phosphates.

### Preservative

In certain embodiments, the functional ingredient is at least one preservative.

As used herein, the at least one preservative may be single preservative or a plurality of preservatives as a functional ingredient for the compositions provided herein. Generally, according to particular embodiments of this invention, the at least one preservative is present in the composition in an amount sufficient to promote health and wellness.

In particular embodiments of this invention, the preservative is chosen from antimicrobials, antioxidants, antienzymatics or combinations thereof. Non-limiting examples of antimicrobials include sulfites, propionates, benzoates, sorbates, nitrates, nitrites, bacteriocins, salts, sugars, acetic acid, dimethyl dicarbonate (DMDC), ethanol, and ozone.

According to a particular embodiment, the preservative is a sulfite. Sulfites include, but are not limited to, sulfur dioxide, sodium bisulfite, and potassium hydrogen sulfite.

According to another particular embodiment, the preservative is a propionate. Propionates include, but are not limited to, propionic acid, calcium propionate, and sodium propionate.

According to yet another particular embodiment, the preservative is a benzoate. Benzoates include, but are not limited to, sodium benzoate and benzoic acid.

In another particular embodiment, the preservative is a sorbate. Sorbates include, but are not limited to, potassium sorbate, sodium sorbate, calcium sorbate, and sorbic acid.

In still another particular embodiment, the preservative is a nitrate and/or a nitrite. Nitrates and nitrites include, but are not limited to, sodium nitrate and sodium nitrite.

In yet another particular embodiment, the at least one preservative is a bacteriocin, such as, for example, nisin.

In another particular embodiment, the preservative is ethanol.

In still another particular embodiment, the preservative is ozone.

Non-limiting examples of antienzymatics suitable for use as preservatives in particular embodiments of the invention include ascorbic acid, citric acid, and metal chelating agents such as ethylenediaminetetraacetic acid (EDTA).

### Hydration Agent

In certain embodiments, the functional ingredient is at least one hydration agent.

As used herein, the at least one hydration agent may be single hydration agent or a plurality of hydration agents as a functional ingredient for the compositions provided herein. Generally, according to particular embodiments of this invention, the at least one hydration agent is present in the composition in an amount sufficient to promote health and wellness.

Hydration products help the body to replace fluids that are lost through excretion. For example, fluid is lost as sweat in order to regulate body temperature, as urine in order to excrete waste substances, and as water vapor in order to exchange gases in the lungs. Fluid loss can also occur due to a wide range of external causes, non-limiting examples of which include physical activity, exposure to dry air, diarrhea, vomiting, hyperthermia, shock, blood loss, and hypotension. Diseases causing fluid loss include diabetes, cholera, gastroenteritis, shigellosis, and yellow fever. Forms of malnutrition that cause fluid loss include the excessive consumption of alcohol, electrolyte imbalance, fasting, and rapid weight loss.

In a particular embodiment, the hydration product is a composition that helps the body replace fluids that are lost during exercise. Accordingly, in a particular embodiment, the hydration product is an electrolyte, non-limiting examples of which include sodium, potassium, calcium, magnesium, chloride, phosphate, bicarbonate, and combinations thereof. Suitable electrolytes for use in particular embodiments of this invention are also described in U.S. Patent No. 5,681,569. In particular embodiments, the electrolytes are obtained from their corresponding water-soluble salts. Non-limiting examples of salts for use in particular embodiments include chlorides, carbonates, sulfates, acetates, bicarbonates, citrates, phosphates, hydrogen phosphates, tartrates, sorbates, citrates, benzoates, or combinations thereof. In other embodiments, the electrolytes are provided by juice, fruit extracts, vegetable extracts, tea, or teas extracts.

In particular embodiments of this invention, the hydration product is a carbohydrate to supplement energy stores burned by muscles. Suitable carbohydrates for use in particular embodiments of this invention are described in U.S. Patent Numbers 4,312,856, 4,853,237, 5,681,569, and 6,989,171. Non-limiting examples of suitable carbohydrates include monosaccharides, disaccharides, oligosaccharides, complex polysaccharides or combinations thereof. Non-limiting examples of suitable types of monosaccharides for use in particular embodiments include trioses, tetroses, pentoses, hexoses, heptoses, octoses, and nonoses. Non-limiting examples of specific types of suitable monosaccharides include glyceraldehyde, dihydroxyacetone, erythrose, threose, erythrulose, arabinose, lyxose, ribose, xylose, ribulose, xylulose, allose, altrose, galactose, glucose, gulose, idose, mannose, talose, fructose, psicose, sorbose, tagatose, mannoheptulose, sedoheltulose, octolose, and sialose. Non-limiting examples of suitable disaccharides include sucrose, lactose, and maltose. Non-limiting examples of suitable oligosaccharides include saccharose, maltotriose, and maltodextrin. In other particular embodiments, the carbohydrates are provided by a corn syrup, a beet sugar, a cane sugar, a juice, or a tea.

In another particular embodiment, the hydration is a flavanol that provides cellular rehydration. Flavanols are a class of natural substances present in plants, and generally comprise a 2-phenylbenzopyrone molecular skeleton attached to one or more chemical moieties. Nonlimiting examples of suitable flavanols for use in particular embodiments of this invention include catechin, epicatechin, gallocatechin, epigallocatechin, epicatechin gallate, epigallocatechin 3-gallate, theaflavin, theaflavin 3-gallate, theaflavin 3'-gallate, theaflavin 3,3' gallate, thearubigin or combinations thereof. Several common sources of flavanols include tea plants, fruits, vegetables, and flowers. In preferred embodiments, the flavanol is extracted from green tea.

In a particular embodiment, the hydration product is a glycerol solution to enhance exercise endurance. The ingestion of a glycerol containing solution has been shown to provide beneficial physiological effects, such as expanded blood volume, lower heart rate, and lower rectal temperature.

### Probiotics/Prebiotics

In certain embodiments, the functional ingredient is chosen from at least one probiotic, prebiotic and combination thereof.

As used herein, the at least one probiotic or prebiotic may be single probiotic or prebiotic or a plurality of probiotics or prebiotics as a functional ingredient for the compositions provided herein. Generally, according to particular embodiments of this invention, the at least one probiotic, prebiotic or combination thereof is present in the composition in an amount sufficient to promote health and wellness.

Probiotics, in accordance with the teachings of this invention, comprise microorganisms that benefit health when consumed in an effective amount. Desirably, probiotics beneficially affect the human body's naturally-occurring gastrointestinal microflora and impart health benefits apart from nutrition. Probiotics may include, without limitation, bacteria, yeasts, and fungi.

Prebiotics, in accordance with the teachings of this invention, are compositions that promote the growth of beneficial bacteria in the intestines. Prebiotic substances can be consumed by a relevant probiotic, or otherwise assist in keeping the relevant probiotic alive or stimulate its growth. When consumed in an effective amount, prebiotics also beneficially affect the human body's naturally-occurring gastrointestinal microflora and thereby impart health benefits apart from just nutrition. Prebiotic foods enter the colon and serve as substrate for the endogenous bacteria, thereby indirectly providing the host with energy, metabolic substrates, and essential micronutrients. The body's digestion and absorption of prebiotic foods is dependent upon bacterial metabolic activity, which salvages energy for the host from nutrients that escaped digestion and absorption in the small intestine.

According to particular embodiments, the probiotic is a beneficial microorganism that beneficially affects the human body's naturally-occurring gastrointestinal microflora and imparts health benefits apart from nutrition. Examples of probiotics include, but are not limited to, bacteria of the genus *Lactobacilli, Bifidobacteria, Streptococci,* or combinations thereof, that confer beneficial effects to humans.

In particular embodiments of the invention, the at least one probiotic is chosen from the genus *Lactobacilli. Lactobacilli* (i.e., bacteria of the genus *Lactobacillus,* hereinafter "*L*.") have been used for several hundred years as a food preservative and for promoting human health. Non-limiting examples of species *of Lactobacilli* found in the human intestinal tract include *L. acidophilus, L. casei, L. fermentum, L. saliva roes, L. brevis, L. leichmannii, L. plantarum, L. cellobiosus, L. reuteri, L. rhamnosus, L. GG, L. bulgaricus,* and *L. thermophilus, .*

According to other particular embodiments of this invention, the probiotic is chosen from the genus *Bifidobacteria. Bifidobacteria* also are known to exert a beneficial influence on human health by producing short chain fatty acids (e.g., acetic, propionic, and butyric acids), lactic, and formic acids as a result of carbohydrate metabolism. Non-limiting species of *Bifidobacteria* found in the human gastrointestinal tract include *B. angulatum, B. animalis, B. asteroides, B. bifidum, B. boum, B. breve, B. catenulatum, B. choerinum, B. coryneforme, B. cuniculi, B. dentium, B. gallicum, B. gallinarum, B indicum, B. longum, B. magnum, B. merycicum, B. minimum, B. pseudocatenulatum, B. pseudolongum, B. psychraerophilum, B. pullorum, B. ruminantium, B. saeculare, B. scardovii, B. simiae, B. subtile, B. thermacidophilum, B. thermophilum, B. urinalis, and B. sp.*

According to other particular embodiments of this invention, the probiotic is chosen from the genus *Streptococcus. Streptococcus thermophilus* is a gram-positive facultative anaerobe. It is classified as a lactic acid bacteria and commonly is found in milk and milk products, and is used in the production of yogurt. Other non-limiting probiotic species of this bacteria include *Streptococcus salivarus* and *Streptococcus cremoris.*

Probiotics that may be used in accordance with this invention are well-known to those of skill in the art. General examples of foodstuffs comprising probiotics include yogurt, sauerkraut, kefir, kimchi, fermented vegetables, and other foodstuffs containing a microbial element that beneficially affects the host animal by improving the intestinal microbalance.

Prebiotics, in accordance with the embodiments of this invention, include, without limitation, mucopolysaccharides, oligosaccharides, polysaccharides, amino acids, vitamins, nutrient precursors, proteins and combinations thereof.

According to a particular embodiment of this invention, the prebiotic is chosen from dietary fibers, including, without limitation, polysaccharides and oligosaccharides. These compounds have the ability to increase the number of probiotics, which leads to the benefits conferred by the probiotics. Non-limiting examples of oligosaccharides that are categorized as prebiotics in accordance with particular embodiments of this invention include fructooligosaccharides, inulins, isomalto-oligosaccharides, lactilol, lactosucrose, lactulose, pyrodextrins, soy oligosaccharides, transgalacto-oligosaccharides, and xylo-oligosaccharides.

According to other particular embodiments of the invention, the prebiotic is an amino acid. Although a number of known prebiotics break down to provide carbohydrates for probiotics, some probiotics also require amino acids for nourishment.

Prebiotics are found naturally in a variety of foods including, without limitation, bananas, berries, asparagus, garlic, wheat, oats, barley (and other whole grains), flaxseed, tomatoes, Jerusalem artichoke, onions and chicory, greens (e.g., dandelion greens, spinach, collard greens, chard, kale, mustard greens, turnip greens), and legumes (e.g., lentils, kidney beans, chickpeas, navy beans, white beans, black beans).

### Weight Management Agent

In certain embodiments, the functional ingredient is at least one weight management agent.

As used herein, the at least one weight management agent may be single weight management agent or a plurality of weight management agents as a functional ingredient for the compositions provided herein. Generally, according to particular embodiments of this invention, the at least one weight management agent is present in the composition in an amount sufficient to promote health and wellness.

As used herein, "a weight management agent" includes an appetite suppressant and/or a thermogenesis agent. As used herein, the phrases "appetite suppressant", "appetite satiation compositions", "satiety agents", and "satiety ingredients" are synonymous. The phrase "appetite suppressant" describes macronutrients, herbal extracts, exogenous hormones, anorectics, anorexigenics, pharmaceutical drugs, and combinations thereof, that when delivered in an effective amount, suppress, inhibit, reduce, or otherwise curtail a person's appetite. The phrase "thermogenesis agent" describes macronutrients, herbal extracts, exogenous hormones, anorectics, anorexigenics, pharmaceutical drugs, and combinations thereof, that when delivered in an effective amount, activate or otherwise enhance a person's thermogenesis or metabolism.

Suitable weight management agents include macronutrient selected from the group consisting of proteins, carbohydrates, dietary fats, and combinations thereof. Consumption of proteins, carbohydrates, and dietary fats stimulates the release of peptides with appetite-suppressing effects. For example, consumption of proteins and dietary fats stimulates the release of the gut hormone cholecytokinin (CCK), while consumption of carbohydrates and dietary fats stimulates release of Glucagon-like peptide 1 (GLP-1).

Suitable macronutrient weight management agents also include carbohydrates. Carbohydrates generally comprise sugars, starches, cellulose and gums that the body converts into glucose for energy. Carbohydrates often are classified into two categories, digestible carbohydrates (e.g., monosaccharides, disaccharides, and starch) and non-digestible carbohydrates (e.g., dietary fiber). Studies have shown that non-digestible carbohydrates and complex polymeric carbohydrates having reduced absorption and digestibility in the small intestine stimulate physiologic responses that inhibit food intake. Accordingly, the carbohydrates embodied herein desirably comprise non-digestible carbohydrates or carbohydrates with reduced digestibility. Non-limiting examples of such carbohydrates include polydextrose; inulin; monosaccharide-derived polyols such as erythritol, mannitol, xylitol, and sorbitol; disaccharidederived alcohols such as isomalt, lactitol, and maltitol; and hydrogenated starch hydrolysates. Carbohydrates are described in more detail herein below.

In another particular embodiment weight management agent is a dietary fat. Dietary fats are lipids comprising combinations of saturated and unsaturated fatty acids. Polyunsaturated fatty acids have been shown to have a greater satiating power than mono-unsaturated fatty acids. Accordingly, the dietary fats embodied herein desirably comprise poly-unsaturated fatty acids, non-limiting examples of which include triacylglycerols.

In a particular embodiment, the weight management agent is an herbal extract. Extracts from numerous types of plants have been identified as possessing appetite suppressant properties. Non-limiting examples of plants whose extracts have appetite suppressant properties include plants of the genus *Hoodia, Trichocaulon, Caralluma, Stapelia, Orbea, Asclepias,* and *Camelia.* Other embodiments include extracts derived from Gymnema Sylvestre, Kola Nut, Citrus Auran tium, Yerba Mate, Griffonia Simplicifolia, Guarana, myrrh, guggul Lipid, and black current seed oil.

The herbal extracts may be prepared from any type of plant material or plant biomass. Non-limiting examples of plant material and biomass include the stems, roots, leaves, dried powder obtained from the plant material, and sap or dried sap. The herbal extracts generally are prepared by extracting sap from the plant and then spray-drying the sap. Alternatively, solvent extraction procedures may be employed. Following the initial extraction, it may be desirable to further fractionate the initial extract (e.g., by column chromatography) in order to obtain an herbal extract with enhanced activity. Such techniques are well known to those of ordinary skill in the art.

In a particular embodiment, the herbal extract is derived from a plant of the genus *Hoodia,* species of which include *H. alstonii, H. currorii, H. dregei, H. flava, H. gordonii, H. jutatae, H. mossamedensis, H. officinalis, H. parviflorai, H. pedicellata, H. pilifera, H. ruschii,* and *H. triebneri. Hoodia* plants are stem succulents native to southern Africa. A sterol glycoside of *Hoodia,* known as P57, is believed to be responsible for the appetite-suppressant effect of the *Hoodia* species.

In another particular embodiment, the herbal extract is derived from a plant of the genus *Caralluma,* species of which include C. *indica, C. fimbriata, C. attenuate, C. tuberculata, C. edulis, C. adscendens, C. stalagmifera, C. umbellate, C. penicillata, C. russeliana, C. retrospicens, C. Arabica,* and C. *lasiantha. Carralluma* plants belong to the same Subfamily as *Hoodia,* Asclepiadaceae. *Caralluma* are small, erect and fleshy plants native to India having medicinal properties, such as appetite suppression, that generally are attributed to glycosides belonging to the pregnane group of glycosides, non-limiting examples of which include caratuberside A, caratuberside B, bouceroside I, bouceroside II, bouceroside III, bouceroside IV, bouceroside V, bouceroside VI, bouceroside VII, bouceroside VIII, bouceroside IX, and bouceroside X.

In another particular embodiment, the at least one herbal extract is derived from a plant of the genus *Trichocaulon. Trichocaulon* plants are succulents that generally are native to southern Africa, similar to *Hoodia,* and include the species *T. piliferum* and *T. officinale.*

In another particular embodiment, the herbal extract is derived from a plant of the genus *Stapelia* or *Orbea,* species of which include *S. gigantean* and O. *variegate,* respectively. Both *Stapelia* and *Orbea* plants belong to the same Subfamily as *Hoodia,* Asclepiadaceae. Not wishing to be bound by any theory, it is believed that the compounds exhibiting appetite suppressant activity are saponins, such as pregnane glycosides, which include stavarosides A, B, C, D, E, F, G, H, I, J, and K.

In another particular embodiment, the herbal extract is derived from a plant of the genus *Asclepias. Asclepias* plants also belong to the Asclepiadaceae family of plants. Non-limiting examples of *Asclepias* plants include *A. incarnate, A. curassayica, A. syriaca,* and *A. tuberose.* Not wishing to be bound by any theory, it is believed that the extracts comprise steroidal compounds, such as pregnane glycosides and pregnane aglycone, having appetite suppressant effects.

In a particular embodiment, the weight management agent is an exogenous hormone having a weight management effect. Non-limiting examples of such hormones include CCK, peptide YY, ghrelin, bombesin and gastrin-releasing peptide (GRP), enterostatin, apolipoprotein A-IV, GLP-1, amylin, somastatin, and leptin.

In another embodiment, the weight management agent is a pharmaceutical drug. Non-limiting examples include phentenime, diethylpropion, phendimetrazine, sibutramine,
rimonabant, oxyntomodulin, floxetine hydrochloride, ephedrine, phenethylamine, or other stimulants.

### Osteoporosis Management Agent

In certain embodiments, the functional ingredient is at least one osteoporosis management agent.

As used herein, the at least one osteoporosis management agent may be single osteoporosis management agent or a plurality of osteoporosis management agent as a functional ingredient for the compositions provided herein. Generally, according to particular embodiments of this invention, the at least one osteoporosis management agent is present in the composition in an amount sufficient to promote health and wellness.

In certain embodiments, the osteoporosis management agent is at least one calcium source. According to a particular embodiment, the calcium source is any compound containing calcium, including salt complexes, solubilized species, and other forms of calcium. Non-limiting examples of calcium sources include amino acid chelated calcium, calcium carbonate, calcium oxide, calcium hydroxide, calcium sulfate, calcium chloride, calcium phosphate, calcium hydrogen phosphate, calcium dihydrogen phosphate, calcium citrate, calcium malate, calcium citrate malate, calcium gluconate, calcium tartrate, calcium lactate, solubilized species thereof, and combinations thereof.

According to a particular embodiment, the osteoporosis management agent is a magnesium soucrce. The magnesium source is any compound containing magnesium, including salt complexes, solubilized species, and other forms of magnesium. Non-limiting examples of magnesium sources include magnesium chloride, magnesium citrate, magnesium gluceptate, magnesium gluconate, magnesium lactate, magnesium hydroxide, magnesium picolate, magnesium sulfate, solubilized species thereof, and mixtures thereof. In another particular embodiment, the magnesium source comprises an amino acid chelated or creatine chelated magnesium.

In other embodiments, the osteoporosis agent is chosen from vitamins D, C, K, their precursors and/or beta-carotene and combinations thereof.

Numerous plants and plant extracts also have been identified as being effective in the prevention and treatment of osteoporosis. Not wishing to be bound by any theory, it is believed that the plants and plant extracts stimulates bone morphogenic proteins and/or inhibits bone resorption, thereby stimulating bone regeneration and strength. Non-limiting examples of suitable plants and plant extracts as osteoporosis management agents include species of the genus *Taraxacum* and *Amelanchier,* as disclosed in U.S. Patent Publication No. 2005/0106215, and species of the genus *Lindera, Artemisia, Acorus, Carthamus, Carum, Cnidium, Curcuma, Cyperus, Juniperus, Prunus, Iris, Cichorium, Dodonaea, Epimedium, Erigonoum, Soya, Mentha, Ocimum, thymus, Tanacetum, Plantago, Spearmint, Bixa, Vitis, Rosemarinus, Rhus,* and *Anethum,* as disclosed in U.S. Patent Publication No. 2005/0079232.

### Phytoestrogen

In certain embodiments, the functional ingredient is at least one phytoestrogen.

As used herein, the at least one phytoestrogen may be single phytoestrogen or a plurality of phytoestrogens as a functional ingredient for the compositions provided herein. Generally, according to particular embodiments of this invention, the at least one phytoestrogen is present in the composition in an amount sufficient to promote health and wellness.

Phytoestrogens are compounds found in plants which can typically be delivered into human bodies by ingestion of the plants or the plant parts having the phytoestrogens. As used herein, "phytoestrogen" refers to any substance which, when introduced into a body causes an estrogen-like effect of any degree. For example, a phytoestrogen may bind to estrogen receptors within the body and have a small estrogen-like effect.

Examples of suitable phytoestrogens for embodiments of this invention include, but are not limited to, isoflavones, stilbenes, lignans, resorcyclic acid lactones, coumestans, coumestroI, equol, and combinations thereof. Sources of suitable phytoestrogens include, but are not limited to, whole grains, cereals, fibers, fruits, vegetables, black cohosh, agave root, black currant, black haw, chasteberries, cramp bark, dong quai root, devil's club root, false unicorn root, ginseng root, groundsel herb, licorice, liferoot herb, motherwort herb, peony root, raspberry leaves, rose family plants, sage leaves, sarsaparilla root, saw palmetto berried, wild yam root, yarrow blossoms, legumes, soybeans, soy products (e.g., miso, soy flour, soymilk, soy nuts, soy protein isolate, tempen, or tofu) chick peas, nuts, lentils, seeds, clover, red clover, dandelion leaves, dandelion roots, fenugreek seeds, green tea, hops, red wine, flaxseed, garlic, onions, linseed, borage, butterfly weed, caraway, chaste tree, vitex, dates, dill, fennel seed, gotu kola, milk thistle, pennyroyal, pomegranates, southernwood, soya flour, tansy, and root of the kudzu vine (pueraria root) and the like, and combinations thereof.

Isoflavones belong to the group of phytonutrients called polyphenols. In general, polyphenols (also known as "polyphenolics"), are a group of chemical substances found in plants, characterized by the presence of more than one phenol group per molecule.

Suitable phytoestrogen isoflavones in accordance with embodiments of this invention include genistein, daidzein, glycitein, biochanin A, formononetin, their respective naturally occurring glycosides and glycoside conjugates, matairesinol, secoisolariciresinol, enterolactone, enterodiol, textured vegetable protein, and combinations thereof.

Suitable sources of isoflavones for embodiments of this invention include, but are not limited to, soy beans, soy products, legumes, alfalfa sprouts, chickpeas, peanuts, and red clover.

### Long-Chain Primary Aliphatic Saturated Alcohol

In certain embodiments, the functional ingredient is at least one long chain primary aliphatic saturated alcohol.

As used herein, the at least one long chain primary aliphatic saturated alcohol may be single long chain primary aliphatic saturated alcohol or a plurality of long chain primary aliphatic saturated alcohols as a functional ingredient for the compositions provided herein. Generally, according to particular embodiments of this invention, the at least one long chain primary aliphatic saturated alcohol is present in the composition in an amount sufficient to promote health and wellness.

Long-chain primary aliphatic saturated alcohols are a diverse group of organic compounds. The term alcohol refers to the fact these compounds feature a hydroxyl group (-OH) bound to a carbon atom. The term primary refers to the fact that in these compounds the carbon atom which is bound to the hydroxyl group is bound to only one other carbon atom. The term saturated refers to the fact that these compounds feature no carbon to carbon pi bonds. The term aliphatic refers to the fact that the carbon atoms in these compounds are joined together in straight or branched chains rather than in rings. The term long-chain refers to the fact that the number of carbon atoms in these compounds is at least 8 carbons).

Non-limiting examples of particular long-chain primary aliphatic saturated alcohols for use in particular embodiments of the invention include the 8 carbon atom 1-octanol, the 9 carbon 1-nonanol, the 10 carbon atom 1-decanol, the 12 carbon atom 1-dodecanol, the 14 carbon atom 1-tetradecanol, the 16 carbon atom 1-hexadecanol, the 18 carbon atom 1-octadecanol, the 20 carbon atom l-eicosanol, the 22 carbon 1-docosanol, the 24 carbon 1-tetracosanol, the 26 carbon 1-hexacosanol, the 27 carbon 1-heptacosanol, the 28 carbon 1-octanosol, the 29 carbon 1- nonacosanol, the 30 carbon 1-triacontanol, the 32 carbon 1-dotriacontanol, and the 34 carbon 1- tetracontanol.

In a particularly desirable embodiment of the invention, the long-chain primary aliphatic saturated alcohols are policosanol. Policosanol is the term for a mixture of long-chain primary aliphatic saturated alcohols composed primarily of 28 carbon 1-octanosol and 30 carbon 1- triacontanol, as well as other alcohols in lower concentrations such as 22 carbon 1-docosanol, 24 carbon 1-tetracosanol, 26 carbon 1-hexacosanol, 27 carbon 1-heptacosanol, 29 carbon 1- nonacosanol, 32 carbon 1-dotriacontanol, and 34 carbon 1-tetracontanol.

Long-chain primary aliphatic saturated alcohols are derived from natural fats and oils. They may be obtained from these sources by using extraction techniques well known to those of ordinary skill in the art. Policosanols can be isolated from a variety of plants and materials including sugar cane *(Saccharum officinarium),* yams *(e.g. Dioscorea opposite),* bran from rice *(e.g. Oryza sativa),* and beeswax. Policosanols may be obtained from these sources by using extraction techniques well known to those of ordinary skill in the art. A description of such extraction techniques can be found in U.S. Pat. Appl. No. 2005/0220868*.*

### Phytosterols

In certain embodiments, the functional ingredient is at least one phytosterol, phytostanol or combination thereof.

Generally, according to particular embodiments of this invention, the at least one phytosterol, phytostanol or combination thereof is present in the composition in an amount sufficient to promote health and wellness.

As used herein, the phrases "stanol", "plant stanol" and "phytostanol" are synonymous.

Plant sterols and stanols are present naturally in small quantities in many fruits, vegetables, nuts, seeds, cereals, legumes, vegetable oils, bark of the trees and other plant sources. Although people normally consume plant sterols and stanols every day, the amounts consumed are insufficient to have significant cholesterol-lowering effects or other health benefits. Accordingly, it would be desirable to supplement food and beverages with plant sterols and stanols.

Sterols are a subgroup of steroids with a hydroxyl group at C-3. Generally, phytosterols have a double bond within the steroid nucleus, like cholesterol; however, phytosterols also may comprise a substituted side chain (R) at C-24, such as an ethyl or methyl group, or an additional double bond. The structures of phytosterols are well known to those of skill in the art.

At least 44 naturally-occurring phytosterols have been discovered, and generally are derived from plants, such as corn, soy, wheat, and wood oils; however, they also may be produced synthetically to form compositions identical to those in nature or having properties similar to those of naturally-occurring phytosterols. According to particular embodiments of this invention, non-limiting examples of phytosterols well known to those or ordinary skill in the art include 4-desmethylsterols (e.g., β-sitosterol, campesterol, stigmasterol, brassicasterol, 22- dehydrobrassicasterol, and Δ5-avenasterol), 4-monomethyl sterols, and 4,4-dimethyl sterols (triterpene alcohols) (e.g., cycloartenol, 24-methylenecycloartanol, and cyclobranol).

As used herein, the phrases "stanol", "plant stanol" and "phytostanol" are synonymous. Phytostanols are saturated sterol alcohols present in only trace amounts in nature and also may be synthetically produced, such as by hydrogenation of phytosterols. According to particular embodiments of this invention, non-limiting examples of phytostanols include β-sitostanol, campestanol, cycloartanol, and saturated forms of other triterpene alcohols.

Both phytosterols and phytostanols, as used herein, include the various isomers such as the α and β isomers (e.g., α-sitosterol and β-sitostanol, which comprise one of the most effective phytosterols and phytostanols, respectively, for lowering serum cholesterol in mammals).

The phytosterols and phytostanols of the present invention also may be in their ester form. Suitable methods for deriving the esters of phytosterols and phytostanols are well known to those of ordinary skill in the art, and are disclosed in U.S. Patent Numbers 6,589,588, 6,635,774, 6,800,317, and U.S. Patent Publication Number 2003/0045473. Non-limiting examples of suitable phytosterol and phytostanol esters include sitosterol acetate, sitosterol oleate, stigmasterol oleate, and their corresponding phytostanol esters. The phytosterols and phytostanols of the present invention also may include their derivatives.

Generally, the amount of functional ingredient in the composition varies widely depending on the particular composition and the desired functional ingredient. Those of ordinary skill in the art will readily ascertain the appropriate amount of functional ingredient for each composition.

### VI. Methods

Methods of enhancing the sweetness of a beverage, and optionally modulating one or more taste attributes of the sweetener, to make the beverage taste more like a sucrose-sweetened beverage are provided.

The invention provides a method of enhancing the sweetness of a beverage comprising (i) providing a beverage comprising a sweetener comprising a sweetening amount of rebaudioside M and/or a sweetening amount of siamenoside I and (ii) adding at least one flavonoid to the beverage to provide a beverage with enhanced sweetness, wherein the at least one flavonoid is one of the following compounds:

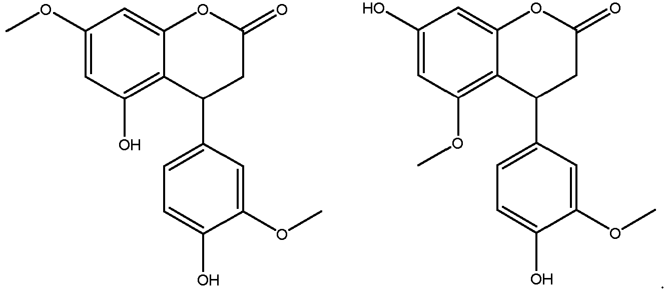

In a particular embodiment, the SE of the beverage comprising the at least one flavonoid and at least one sweetener is enhanced by at least about 1.2-fold compared to the SE of the beverage in the absence of the at least one flavonoid, such as, for example, at least about 1.3-fold, at least about 1.4-fold, at least about 1.5-fold, at least about 1.6-fold, at least about 1.7- fold, at least about 1.8-fold, at least about 1.9-fold and at least about 2.0-fold.

As discussed above, in the present methods the at least one flavonoid may be present in the beverage in the identified concentrations. Methods for preparing beverages with enhanced sweetness are also provided.

### EXAMPLES

### EXAMPLE 1: Sweetness Enhancement of Rebaudioside M

The ability of the following compounds to enhance the sweetness of Rebaudioside M was evaluated:

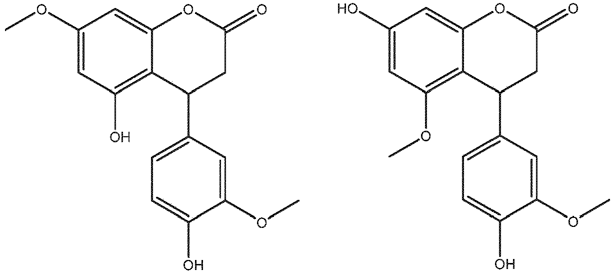

### Test mixture 1 (mixture of regioisomers)

### Sample Preparation

Sample were prepared with the following ingredients:

**Table 1: 40 ppm of Test Mixture 1 in acidic water**

| **Ingredient** | **Amount** |
|---|---|
| Test Mixture 1 | 10 mg |
| Propylene glycol | 0.5 g |
| Citric acid | 62.5 mg |
| Water | 249.5 g |

| | |
|---|---|
| * Initially Test Mixture 1 was dissolved in Propylene glycol. Then, water and citric acid were added. Propylene glycol was used to improve the solubility of Test Mixture 1. | |

**Table 2: Acidic water**

| **Ingredient** | **Amount** |
|---|---|
| Propylene glycol | 1 g |
| Citric acid | 125 mg |
| Water | 499 g |

**Table 3: 150 ppm of Rebaudioside M in acidic water**

| **Ingredient** | **Amount** |
|---|---|
| Rebaudioside M | 30 mg |
| Acidic water (Table 2) | 200 g |

**Table 4: 40 ppm of Test Mixture 1 and 150 ppm of Rebaudioside M in acidic water**

| **Ingredient** | **Amount** |
|---|---|
| Rebaudioside M | 30 mg |
| Test Mixture 1 in Water (Table 1) | 200 g |

**Table 5: Sucrose (wt%) in acidic water**

| **Ingredient** | **1.5% Sucrose** | **6% Sucrose** | **8% Sucrose** | **10% Sucrose** |
|---|---|---|---|---|
| Sucrose | 1.5 g | 6 g | 8 g | 10 g |
| Citric acid | 25 mg | 25 mg | 25 mg | 25 mg |
| Water | 98.5 g | 94 g | 92 g | 90 g |

Ingredients were added to water or water with Test Mixture 1 while stirring until solids were visibly dissolved and the sample was poured into a glass bottle and stored at 4°C.

### Taste Evaluation

Trained panelists compared the taste and sweetness profiles of (i) the beverage containing 150 ppm Rebaudioside M (Table 3) and (ii) the beverage containing 40 ppm Test Mixture 1 and 150 ppm Rebaudioside M (Table 4) against the 6%, 8%, and 10% sucrose beverages (Table 5).

Bottles were removed from the refrigerator and about 25 ml of beverage was poured into 4 oz- plastic cups. Panelists were given mineral water to rinse their mouth before and between tastings. Unsalted crackers were also given to panelists to eat followed by rinsing their mouth with mineral water before tasting the next sample. Panelists were instructed to sip, evaluate the sweetness, and then spit the samples in cups provided for that purpose.

Panelists found that the beverage containing 150 ppm Rebaudioside M was as sweet as the 5-6% sucrose beverage. The beverage containing 40 ppm Test Mixture 1 and 150 ppm Rebaudioside M was found to be at least equal to the 9% sucrose beverage and have a sugar-like taste

### EXAMPLE 2: Sweetness Enhancement of Siamenoside I

The ability of Text Mixture 1 to enhance the sweetness of Siamenoside I was evaluated.

### Sample Preparation

Sample were prepared with the following ingredients:

**Table 1: 40 ppm of Test Mixture 1 in acidic water**

| **Ingredient** | **Amount** |
|---|---|
| Test Mixture 1 | 10 mg |
| Propylene glycol | 0.5 g |
| Citric acid | 62.5 mg |
| Water | 249.5 g |

| | |
|---|---|
| * Initially Test Mixture 1 was dissolved in Propylene glycol. Then, water and citric acid were added. Propylene glycol is used to improve the solubility of Test Mixture 1. | |

**Table 2: Acidic water**

| **Ingredient** | **Amount** |
|---|---|
| Propylene glycol | 1 g |
| Citric acid | 125 mg |
| Water | 499 g |

**Table 3: 150 ppm of Siamenoside I in acidic water**

| **Ingredient** | **Amount** |
|---|---|
| Siamenoside I | 30 mg |
| Acidic water (Table 2) | 200 g |

**Table 4: 40 ppm of Test Mixture 1 and 150 ppm of Siamenoside I in acidic water**

| **Ingredient** | **Amount** |
|---|---|
| Siamenoside I | 30 mg |
| Test Mixture 1 in acidic Water (Table 1) | 200 g |

**Table 5: Sucrose (wt%) in acidic water**

| **Ingredient** | **1.5% Sucrose** | **4% Sucrose** | **6% Sucrose** | **8% Sucrose** | **10% Sucrose** |
|---|---|---|---|---|---|
| Sucrose | 1.5 g | 4 g | 6 g | 8 g | 10 g |
| Citric acid | 25 mg | 25 mg | 25 mg | 25 mg | 25 mg |
| Water | 98.5 g | 96 g | 94 g | 92 g | 90 g |

Ingredients were added to water or water with Test Mixture 1 while stirring until solids were visibly dissolved and the sample was poured into a glass bottle and stored at 4°C.

### Taste Evaluation

First, trained panelists assessed the sweetness of 40 ppm Test Mixture 1 (Table 1) against the 1.5% sucrose beverage. Panelists found that 40 ppm Test Mixture 1 had sweetness below the sweetness of less than 1.5% sucrose solution.

The trained panelists then compared the taste and sweetness profiles of (i) the beverage containing 150 ppm Siamenoside I (Table 3) and (ii) the beverage containing 40 ppm Test Mixture 1 and 150 ppm Siamenoside I (Table 4) against the 6%, 8%, and 10% sucrose beverages (Table 5).

Bottles were removed from the refrigerator and about 25 ml of beverage was poured into 4 oz- plastic cups. Panelists were given mineral water to rinse their mouth before and between tastings. Unsalted crackers were also given to panelists to eat followed by rinsing their mouth with mineral water before tasting the next sample. Panelists were instructed to sip, evaluate the sweetness, and then spit the samples in cups provided for that purpose.

Panelists found that the beverage containing 150 ppm Siamenoside I was equivalent to 4%-6% sucrose beverages in terms of sweetness. Panelists reported that the beverage containing 40 ppm Test Mixture 1 and 150 ppm Siamenoside I increased in sweetness equivalency by 2% SE compared to the Siamenoside I-only beverage and was reported to have better mouthfeel, less bitterness and less sweetness linger compared to the Siamenoside I-only beverage.

## Claims

1. A beverage comprising at least one sweetener and at least one flavonoid, wherein the at least one sweetener is selected from a steviol glycoside blend comprising rebaudioside M in a sweetening amount and a mogroside blend comprising siamenoside I in a sweetening amount; and the at least one flavonoid is one of the following compounds:

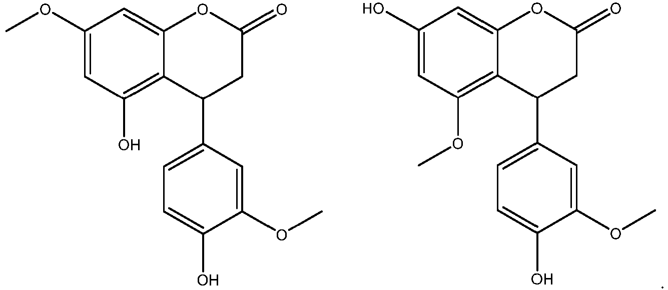

2. The beverage of claim 1, wherein the at least one flavonoid is present in the beverage in a concentration from about 1 ppm to about 50 ppm.

3. The beverage of claim 1, wherein the at least one sweetener is present in the beverage in a concentration from about 50 ppm to about 600 ppm.

4. The beverage of claim 1, wherein when the sweetener is a steviol glycoside blend, the weight ratio of steviol glycosides to at least one flavonoid is greater than about 25:1 or less than about 4:1.

5. The beverage of claim 1, wherein the beverage comprises a mixture of the two flavonoid compounds.

6. A method for enhancing the sweetness of a beverage comprising (i) providing a beverage comprising a sweetener comprising a sweetening amount of rebaudioside M and/or a sweetening amount of siamenoside I and (ii) adding at least one flavonoid to the beverage to provide a beverage with enhanced sweetness, wherein the at least one flavonoid is one of the following compounds:

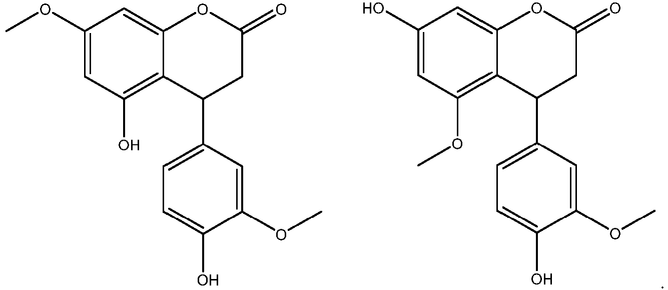

7. The method of claim 6, wherein the flavonoid is added to the beverage in a concentration from about 1 ppm to about 50 ppm.

8. The method of claim 7, wherein the sweetener is present in the beverage in a concentration from about 50 ppm to about 600 ppm.

9. The method of claim 6, wherein step (ii) comprises adding a mixture of the two flavonoid compounds to the beverage.

10. The beverage of any of claims 1-5 or the method of any of claims 6-9, wherein the beverage is selected from the group consisting of a full-calorie, mid-calorie, low-calorie and zero-calorie beverage.

## Patentansprüche

1. Getränk, das mindestens einen Süßstoff und mindestens ein Flavonoid umfasst, wobei der mindestens eine Süßstoff aus einer Steviolglykosidmischung, die Rebaudiosid M in einer Süßungsmenge umfasst, und einer Mogrosidmischung, die Siamenosid I in einer Süßungsmenge umfasst, ausgewählt ist und das mindestens eine Flavonoid eine der folgenden Verbindungen ist:

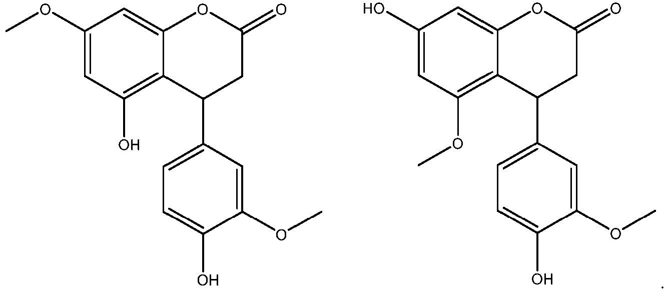

2. Getränk nach Anspruch 1, wobei das mindestens eine Flavonoid in dem Getränk in einer Konzentration von etwa 1 ppm bis etwa 50 ppm vorliegt.

3. Getränk nach Anspruch 1, wobei der mindestens eine Süßstoff in dem Getränk in einer Konzentration von etwa 50 ppm bis etwa 600 ppm vorliegt.

4. Getränk nach Anspruch 1, wobei, wenn der Süßstoff eine Steviolglycosidmischung ist, das Gewichtsverhältnis von Steviolglycosiden zu mindestens einem Flavonoid größer als etwa 25:1 oder kleiner als etwa 4:1 ist.

5. Getränk nach Anspruch 1, wobei das Getränk eine Mischung der beiden Flavonoidverbindungen umfasst.

6. Verfahren zur Verbesserung der Süße eines Getränks, umfassend (i) die Bereitstellung eines Getränks, das einen Süßstoff umfasst, der eine Süßungsmenge an Rebaudiosid M und/oder eine Süßungsmenge an Siamenosid I umfasst, und (ii) das Hinzufügen mindestens eines Flavonoids zu dem Getränk unter Bereitstellung eines Getränks mit verbesserter Süße, wobei das mindestens eine Flavonoid eine der folgenden Verbindungen ist:

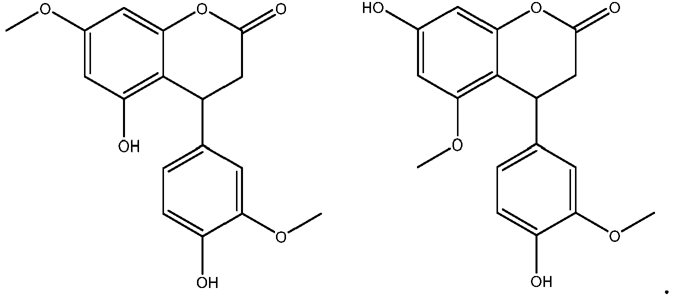

7. Verfahren nach Anspruch 6, wobei das Flavonoid dem Getränk in einer Konzentration von etwa 1 ppm bis etwa 50 ppm zugesetzt wird.

8. Verfahren nach Anspruch 7, wobei der Süßstoff in dem Getränk in einer Konzentration von etwa 50 ppm bis etwa 600 ppm vorliegt.

9. Verfahren nach Anspruch 6, wobei Schritt (ii) die Zugabe einer Mischung der beiden Flavonoidverbindungen zu dem Getränk umfasst.

10. Getränk nach einem der Ansprüche 1-5 oder Verfahren nach einem der Ansprüche 6-9, wobei das Getränk aus der aus einem vollkalorischen, mittelkalorischen, kalorienarmen und kalorienfreien Getränk bestehenden Gruppe ausgewählt ist.

## Revendications

1. Boisson comprenant au moins un édulcorant et au moins un flavonoïde, dans laquelle l'au moins un édulcorant est choisi parmi un mélange de glycosides de stéviol comprenant du rébaudioside M en une quantité édulcorante et un mélange de mogrosides comprenant du siaménoside I en une quantité édulcorante ; et l'au moins un flavonoïde est l'un des composés suivants :

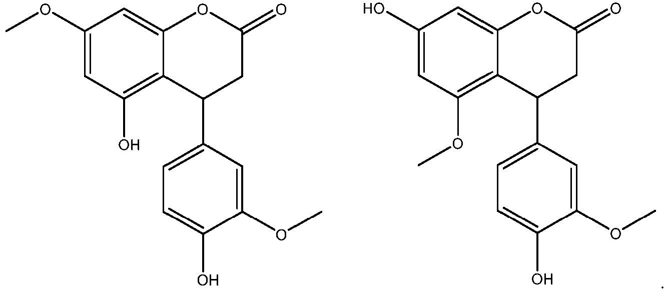

2. Boisson selon la revendication 1, dans laquelle l'au moins un flavonoïde est présent dans la boisson en une concentration d'environ 1 ppm à environ 50 ppm.

3. Boisson selon la revendication 1, dans laquelle l'au moins un édulcorant est présent dans la boisson en une concentration d'environ 50 ppm à environ 600 ppm.

4. Boisson selon la revendication 1, dans laquelle, lorsque l'édulcorant est un mélange de glycosides de stéviol, le rapport pondéral de glycosides de stéviol sur l'au moins un flavonoïde est supérieur à environ 25:1 ou inférieur à environ 4:1.

5. Boisson selon la revendication 1, dans laquelle la boisson comprend un mélange des deux composés flavonoïdes.

6. Procédé pour améliorer la sucrosité d'une boisson comprenant (i) la fourniture d'une boisson comprenant un édulcorant comprenant une quantité édulcorante de rébaudioside M et/ou une quantité édulcorante de siaménoside I et (ii) l'ajout d'au moins un flavonoïde à la boisson pour fournir une boisson ayant une sucrosité améliorée, dans lequel l'au moins un flavonoïde est l'un des composés suivants :

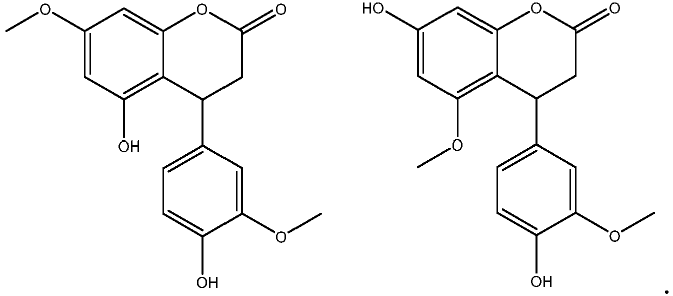

7. Procédé selon la revendication 6, dans lequel le flavonoïde est ajouté à la boisson en une concentration d'environ 1 ppm à environ 50 ppm.

8. Procédé selon la revendication 7, dans lequel l'édulcorant est présent dans la boisson en une concentration d'environ 50 ppm à environ 600 ppm.

9. Procédé selon la revendication 6, dans lequel l'étape (ii) comprend l'ajout d'un mélange des deux composés flavonoïdes à la boisson.

10. Boisson selon l'une quelconque des revendications 1-5 ou procédé selon l'une quelconque des revendications 6 à 9, dans lequel la boisson est choisie dans le groupe constitué par une boisson à teneur calorique normale, moyennement calorique, faiblement calorique et sans calories.
